# EUROPEAN PATENT SPECIFICATION

(11) **EP 4 440 628 B1**
(45) Date of publication and mention of the grant of the patent: **31.12.2025**
(21) Application number: 22823030.6
(22) Date of filing: 29.11.2022
(51) Int. Cl.: A61K 51/08, A61P 35/00

(54) **UROKINASE-TYPE PLASMINOGEN ACTIVATOR RECEPTOR (UPAR)-PET/CT IN HEAD AND NECK SQUAMOUS CELL CARCINOMAS (HNSCCS)**
UROKINASE-TYP-PLASMINOGEN-AKTIVATOR-REZEPTOR (UPAR)-PET/CT IN KOPF- UND NACKENPLATTENEPITHELKARZINOMEN (HNSCCS)
TOMOGRAPHIE/TEP DE RÉCEPTEUR D'ACTIVATEUR DE PLASMINOGÈNE DE TYPE UROKINASE (UPAR)-PET/CT DANS DES CARCINOMES À CELLULES SQUAMEUSES DE LA TÊTE ET DU COU

(30) Priority: 30.11.2021 EP 21211370
(43) Date of publication of application: 09.10.2024
(73) Proprietor: Curasight A/S, 2200 Copenhagen N (DK)
(72) Inventor: KJÆR, Andreas, 2200 København N (DK)
(74) Representative: Plougmann Vingtoft a/s
(86) International application number: PCT/EP2022/083665
(87) International publication number: WO 2023/099472

(56) References cited:
- WO-A1-2014/086364
- RIS�R LOUISE MADELEINE ET AL: "Prognostic value of Urokinase-type Plasminogen Activator Receptor (uPAR)-PET/CT in Head and Neck Squamous Cell Carcinomas and Comparison with 18 F-FDG-PET/CT: A single-center prospective study", THE JOURNAL OF NUCLEAR MEDICINE, 2 December 2021 (2021-12-02), US, pages jnumed.121.262866, XP055914654, ISSN: 0161-5505, Retrieved from the Internet <URL:http://dx.doi.org/10.2967/jnumed.121.262866> DOI: 10.2967/jnumed.121.262866
- ANDERS CHRISTENSEN ET AL: "uPAR-targeted optical near-infrared (NIR) fluorescence imaging and PET for image-guided surgery in head and neck cancer: proof-of-concept in orthotopic xenograft model", ONCOTARGET, vol. 8, no. 9, 28 February 2017 (2017-02-28), pages 15407 - 15419, XP055738185, DOI: 10.18632/oncotarget.14282
- ANONYMOUS: "Phase II Trial: uPAR-PET/CT for Prognostication in Head- and Neck Cancer", CLINICALTRIALS.GOV, 8 October 2020 (2020-10-08), pages 1 - 6, XP055914372, Retrieved from the Internet <URL:https://clinicaltrials.gov/ct2/show/study/NCT02965001> [retrieved on 20220421]
- ANONYMOUS: "uPAR PET/CT for Staging Advanced and Localised Oral and Oropharyngeal Cancer - Tabular View - ClinicalTrials.gov", CLINICALTRIALS.GOV, 10 November 2016 (2016-11-10), pages 1 - 7, XP055914383, Retrieved from the Internet <URL:https://clinicaltrials.gov/ct2/show/record/NCT02960724> [retrieved on 20220421]
- CHRISTENSEN ANDERS ET AL: "Urokinase-type plasminogen activator receptor (uPAR), tissue factor (TF) and epidermal growth factor receptor (EGFR): tumor expression patterns and prognostic value in oral cancer", vol. 17, no. 1, 25 August 2017 (2017-08-25), LONDON, GB, pages 572 - 583, XP055914368, ISSN: 1471-2407, Retrieved from the Internet <URL:https://bmccancer.biomedcentral.com/track/pdf/10.1186/s12885-017-3563-3.pdf> DOI: 10.1186/s12885-017-3563-3
- LOFT MATHIAS DYRBERG ET AL: "Improved positron emission tomography imaging of glioblastoma cancer using novel68Ga-labeled peptides targeting the urokinase-type plasminogen activator receptor (uPAR)", AMINO ACIDS, SPRINGER VERLAG, AU, vol. 49, no. 6, 18 March 2017 (2017-03-18), pages 1089 - 1100, XP036238802, ISSN: 0939-4451, [retrieved on 20170318], DOI: 10.1007/S00726-017-2407-4
- SRINIVAS KONDAVEETISATISH ET AL: "The prognostic role of maximum standardized uptake value of 18 F-flourodeoxy glucose positron emission tomography-computed tomography in head and neck cancer patients undergoing chemoradiotherapy", vol. 9, no. 2, 1 January 2019 (2019-01-01), IN, pages 159, XP055914521, ISSN: 2231-0762, Retrieved from the Internet <URL:https://www.ncbi.nlm.nih.gov/pmc/articles/PMC6489513/pdf/JISPCD-9-159.pdf> DOI: 10.4103/jispcd.JISPCD_409_18
- RISØR LOUISE MADELEINE ET AL: "Urokinase-type plasminogen activator receptor (uPAR) assessed by liquid biopsies and PET/CT for prognostication in head and neck cancer patients", SCIENTIFIC REPORTS, vol. 12, no. 1, 9 November 2022 (2022-11-09), XP093023523, Retrieved from the Internet <URL:https://www.nature.com/articles/s41598-022-21175-7.pdf> DOI: 10.1038/s41598-022-21175-7

## Description

### Technical field of the invention

The present invention relates to a positron-emitting radionuclide labelled peptide conjugate for use in the prognosis of a head and neck cancer (HNSCC) in a patient by PET imaging of the tumor. In particular, the present invention relates to the use of the conjugate 68GA-NOTA-AE105 in the prognosis of relapse-free survival (RFS) for a subject suffering from head and neck cancer (HNSCC) using PET imaging.

### Background of the invention

Traditionally, head and neck squamous cell carcinoma (HNSCC) is caused by alcohol and tobacco, but in recent years a rising incidence of oropharyngeal cancers (OPSCC) has been associated with human papillomavirus (HPV) (1). HPV-positive tumors currently accounts for 63 % of the OPSCC in Western Europe and have a significantly favorable prognosis (2,3). HPV-positive and HPV-negative OPSCC represent distinct molecular and clinical entities and new staging guidelines reduce the stage allocation of HPV-positive tumors based on p16 immunohistochemistry as a surrogate marker of HPV driven carcinogenesis (3,4).

However, recent clinical trials investigating de-escalating treatment regimens in low-risk HPV-positive OPSCC resulted in inferior survival of the de-escalating arms (5-8). To date no reliable method of identifying candidates for de-escalating treatment exists and HPV-positive and negative OPSCC are treated alike (3,9).

Tumor, Node and Metastasis (TNM) stage and HPV are the most important prognostic factors in HNSCC, but besides HPV no prognostic biomarkers are available in clinical practice. Regarding the prognostic value of 18F-FDG, inconsistent results have been published (9-11).

The urokinase-type Plasminogen Activator Receptor (uPAR) promotes cancer cell invasion by degrading the extracellular matrix and facilitates several carcinogenic processes e.g. proliferation and migration (12-14). High uPAR expression has been reported in many cancer types by other means than PET, including HNSCC and has been associated with aggressive disease, distant metastasis and poor survival (*14*). uPAR is located on the cell surface and has limited expression in the surrounding tissue (13). Studies using 68Ga- and 64Cu-labeled AE105-radioligands for uPAR-PET in patients with different cancer types have been performed (15-17), however never in relation to HNSCC.

WO2014/086364 A1 discloses positron-emitting radionuclide labelled peptides for human uPAR PET imaging of cancers, including 68Ga-NOTA-AE105, 68Ga-DOTA-AE105, 64Cu-NOTA-AE105 and 64Cu-DOTA-AE105. WO2014/086364 A1 is silent in respect of HNSCC.

Hence, an improved prognostic method for HNSCC patients would be advantageous.

### Summary of the invention

The aim of the current study was to investigate the prognostic value of uPAR-PET tracers (AE105), (exemplified by 68Ga-NOTA-AE105) in HNSCC patients using PET/CT and to compare it with 18F-FDG-PET.

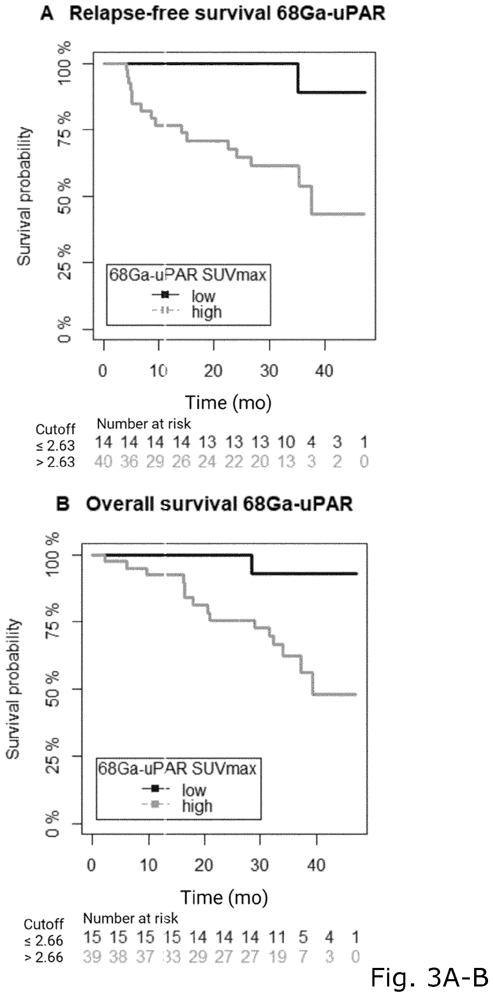

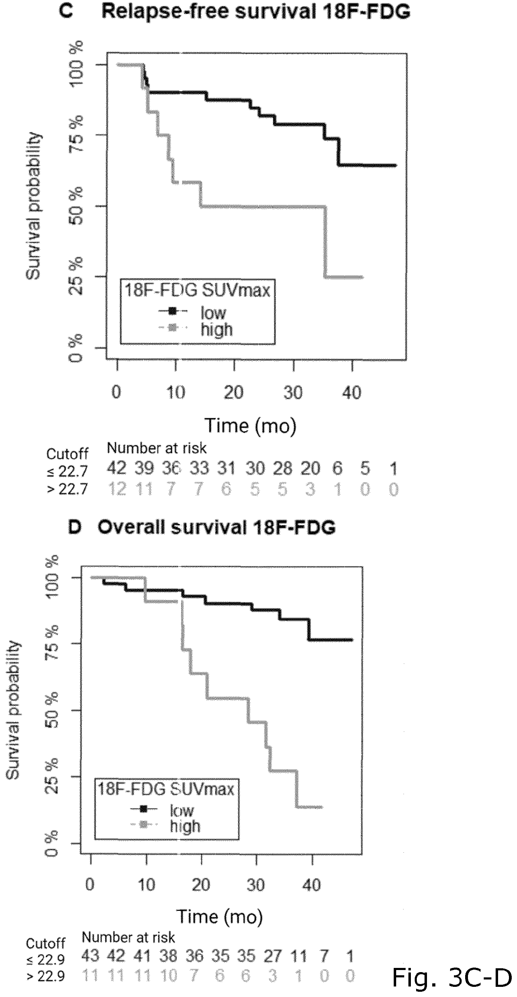

It was found that high primary tumor uptake of the uPAR-PET-tracer was associated with poor relapse-free survival (RFS) in HNSCC patients, whereas low primary tumor uptake of the uPAR-PET-tracer was associated with good relapse-free survival (RFS) in HNSCC patients. In particular, low levels of uPAR performed surprisingly better than 18F-FDG-PET when assessing relapse-free survival (RFS) (See e.g. example 5 and compare Figure 3A and 3C).

Thus, uPAR-PET/CT offers a potential tool for clinicians to select low-risk HNSCC patients for de-escalated treatment regimens to avoid unnecessary toxicity and for a risk stratified follow-up schedule.

Thus, an object of the present invention relates to the provision of improved prognostic tools for HNSCC patients.

In particular, it is an object of the present invention to provide an improved prognostic tools in relation to identifying relapse-free survival (RFS) of HNSCC patients.

Thus, an aspect of the present invention is to provide a positron-emitting imaging agent for use in the prognostication of a head and neck cancer (HNSCC) patient by PET imaging of the cancer,
wherein said imaging agent comprises a uPAR binding peptide coupled via the chelating agent NOTA or DOTA to the radionuclide 68Ga or 64Cu; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof.

The two uPAR-PET tracers 64Cu-DOTA-AE105 and 68Ga-NOTA-AE105 share the same binding moiety, the peptide AE105. In addition, they have both been tested in both humans and animals (e.g. 15, 17 and WO2014/086364 A1) and have demonstrated similar uptake in breast, bladder and prostate cancer. Accordingly, without being bound by theory, it is believed PET tracers comprising
- 64Cu or 68Ga as radionuclide;
- DOTA or NOTA as chelator; and
- the uPAR binding peptide according to the present invention;
will work in a similar manner also for HNSCC patients.

Another aspect of the invention relates to a positron-emitting imaging agent for use in the prognostication of a head and neck cancer (HNSCC) patient by PET imaging of the cancer,
wherein said imaging agent comprises a uPAR binding peptide coupled via the chelating agent NOTA to the radionuclide 68Ga; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) (AE105) or uPAR binding variants thereof.

Yet an aspect relates to a positron-emitting imaging agent for use in the prognostication of relapse-free survival (RFS) and/or overall survival (OS) of a head and neck squamous cell carcinoma (HNSCC) in a human patient by PET imaging of the cancer,
wherein said imaging agent comprises a uPAR binding peptide coupled via the chelating agent NOTA to the radionuclide 68Ga; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof;
wherein the uPAR binding variant is selected from the group consisting of
   - (D-Asp)-([beta]-cyclohexyl-L-alanine)-(Phe)-(D-Ser)-(D-Arg)-(Tyr)-(Leu)-(Trp)-(Ser),
   - (Ser)-(Leu)-([beta]-cyclohexyl-L-alanine)-(Phe)-(D-Ser)-(Gln)-(Tyr)(Leu)-(Trp)-(Ser),
   - (D-Glu)-([beta]-cyclohexyl-L-alanine)-(Phe)-(D-Ser)-(D-Tyr)-(Tyr)-(Leu)-(Trp)-(Ser),
   - (Asp)-([beta]-cyclohexyl-L-alanine)-(Phe)-(D-Ser)-(D-Arg)-(Tyr)-(Leu)-(Trp)-(Ser),
   - (Asp)-([beta]-cyclohexyl-L-alanine)-(Phe)-(Ser)-(D-Arg)-(Tyr)-Leu)-(Trp)-(Ser),
   - (D-Asp)-([beta]-cyclohexyl-L-alanine)-(Phe)-(Ser)-(D-Arg)-(Tyr)-Leu)-(Trp)-(Ser),
   - (D-Thr)-([beta]-cyclohexyl-L-alanine)-(Phe)-(D-Ser)-(D-Arg)-(Tyr)-(Leu)-(Trp)-(Ser),
   - (D-Asp)-([beta]-cyclohexyl-L-alanine)-(Phe)-(D-Ser)-(D-Arg)-(Tyr)-(Leu)-([beta]-2-naphthyl-L-alanine)-(Ser),
   - (Asp)-([beta]-cyclohexyl-L-alanine)-(Phe)-(D-Ser)-(Arg)-(Tyr)-(Leu)-(Trp)-(Ser),
   - (Asp)-([beta]-cyclohexyl-L-alanine)-(Phe)-(D-Ser)-(D-Arg)-(Tyr)-(Leu)([beta]-1-naphthyl-L-alanine)-(Ser),
   - (D-Glu)-([beta]-cyclohexyl-L-alanine)-(Phe)-(D-Ser)-(Tyr)-(Tyr)-(Leu)-(Trp)-(Ser),
   - (Asp)-([beta]-cyclohexyl-L-alanine)-(Phe)-(D-Ser)-(D-Arg)-(Leu)-(Leu)-(Trp)-(D-His),
   - (Asp)-([beta]-cyclohexyl-L-alanine)-(Phe)-(D-Ser)-(D-Arg)-([beta]-cyclohexyl-L-alanine)-(Leu)-(Trp)-(Ile),
   - (Asp)-([beta]-cyclohexyl-L-alanine)-(Phe)-(D-Ser)-(D-Arg)-(Tyr)-(Leu)([beta]-1-naphthyl-L-alanine)-(D-His),
   - (Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
   - (Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
   - (Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine), and
   - (Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(Ile);

- wherein a SUVmax and/or SUVmean quantification level above a threshold level is indicative of a poor Relapse-free survival (RFS) prognosis and/or poor overall survival (OS) prognosis; and
- wherein a SUVmax and/or SUVmean quantification level equal to or below a threshold level is indicative of good Relapse-free survival (RFS) prognosis and/or good overall survival (OS) prognosis.

A further aspect of the present invention relates to a method of in vivo imaging by PET imaging, for assessing the prognosis of a head and neck cancer (HNSCC) in a patient, said method comprising:
a) provision of a subject to which a imaging agent as defined according to the invention has been previously administered;
b) detecting by in vivo PET imaging the radioactive emissions from the 68Ga radioisotope of the administered imaging agent of step a);
c) generating an image representative of the location and/or amount of said radioactive emissions;
d) determining the distribution and extent of uPAR expression in said subject, wherein said expression is correlated with said signals emitted by said in vivo imaging agent; and
e) comparing the determined distribution and extent of uPAR expression to a threshold level;
   ▪ wherein a SUVmax and/or SUVmean quantification level above a threshold level is indicative of a poor Relapse-free survival (RFS) prognosis and/or poor overall survival (OS) prognosis; and
   ▪ wherein a SUVmax and/or SUVmean quantification level equal to or below a threshold level is indicative of good Relapse-free survival (RFS) prognosis and/or good overall survival (OS) prognosis.

Yet another aspect of the present invention relates to the use of a positron-emitting imaging agent according to the invention in the prognosis of head and neck cancer (HNSCC) patients by in vivo PET imaging of uPAR expressing tumors,
wherein said imaging agent comprises a uPAR binding peptide coupled via the chelating agent NOTA to the radionuclide 68Ga;
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof.

### Brief description of the figures

*Figure 1*

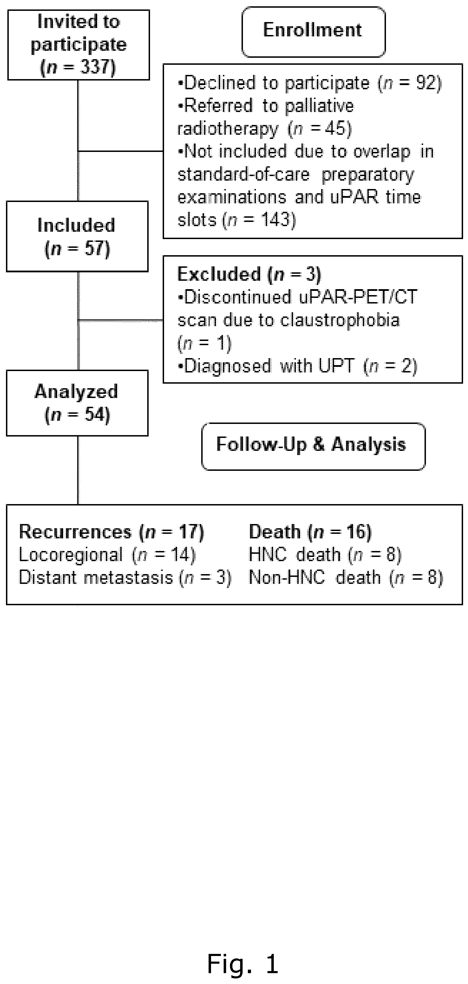

   Figure 1 shows consort flow diagram of inclusion process.
*Figure 2*

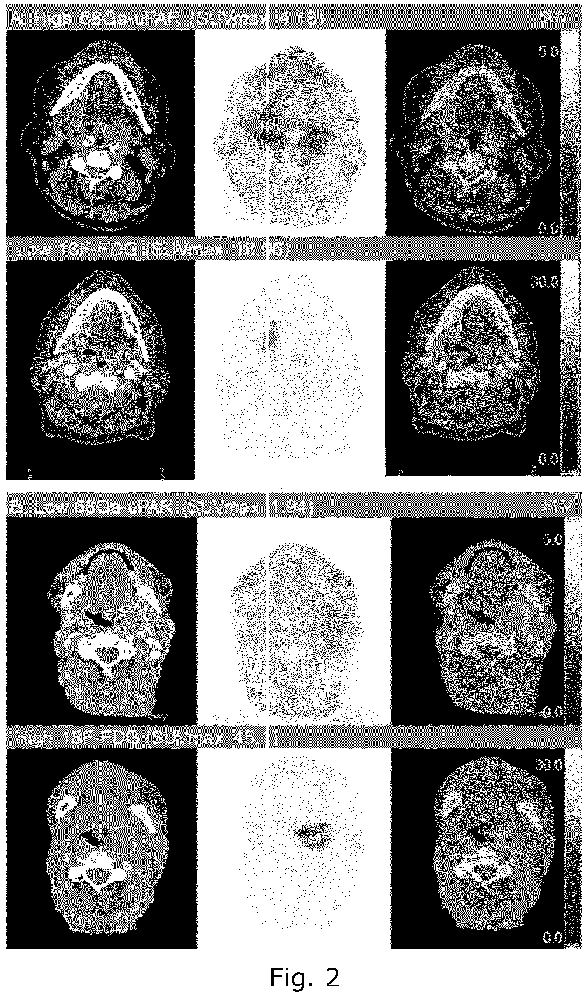

   Figure 2 shows delineated tumor volumes of interest in uPAR-PET/CT with 68Ga-NOTA-AE105 and 18F-FDG-PET/CT in two cases of discordant high 68Ga-uPAR/low 18F-FDG **(A)** and low 68Ga-uPAR/high 18F-FDG **(B).** Both cases present with stage 3 oropharyngeal cancer (T3N0M0). High and low refers to above or below the established cut-offs.
*Figure 3*
   Figure 3 shows Kaplan-Meier plots of **(A)** RFS for 68Ga-uPAR, **(B)** OS for 68Ga-uPAR, **(C)** RFS for 18F-FDG and **(D)** OS for 18F-FDG stratified by the corresponding 68Ga-uPAR- SUVmax cut-offs and 18F-FDG-SUVmax cut-offs.
*Figure 4*

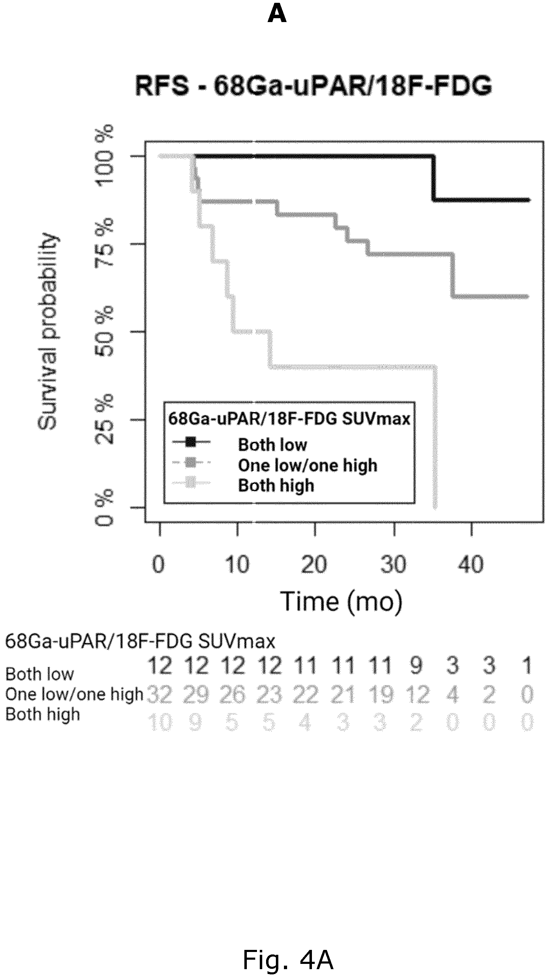

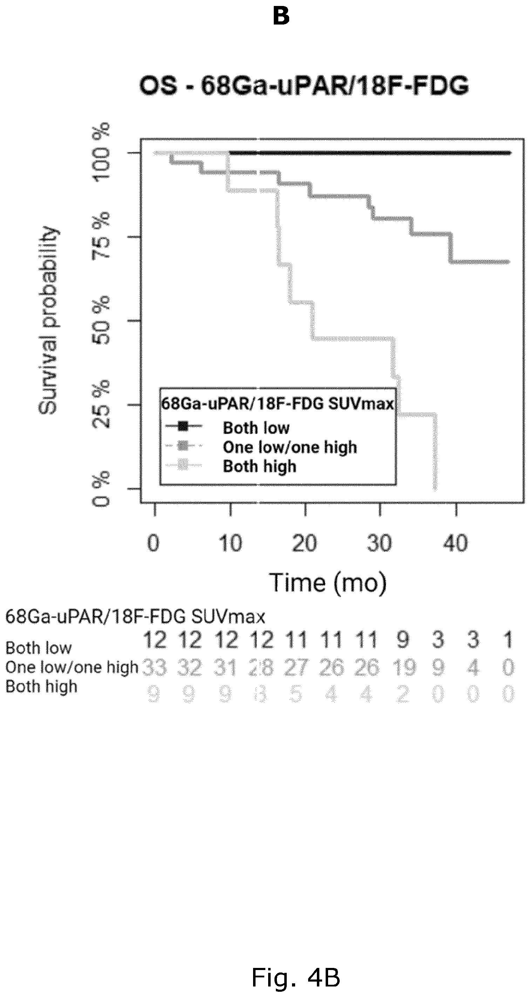

   Figure 4 shows Kaplan-Meier plots of **(A)** Relapse-free survival (RFS) and **(B)** Overall survival (OS) for the concordant and discordant groups; 68Ga-uPAR and 18F-FDG both low (Top curve at end point); one low/one high (Middle curve at end point); and both high (Bottom curve at end point).

The present invention will now be described in more detail in the following.

### Detailed description of the invention

### Definitions

Prior to discussing the present invention in further details, the following terms and conventions will first be defined:

### HNSCC

Head and neck squamous cell carcinomas (HNSCC) are a heterogeneous group of malignant tumours often caused by alcohol and tobacco consumption, although an increasing number of HNSCC arise due to persistent infection with high-risk human papilloma virus (HPV).

In an embodiment, the HNSCC according to the present invention is located in the pharynx, larynx or oral cavity.

### 68Ga

Gallium-68.

### 64Cu

Copper-64.

### AE105

Ac-Asp-Cha-Phe-(D)Ser-(D)Arg-Tyr-Leu-Trp-Ser

The peptide according to the invention can e.g. be synthesized by standard solid-phase peptide chemistry.

### NOTA

NOTA: 2,2',2"-(1,4,7-triazacyclononane-1,4,7-triyl)triacetic acid.

NOTA may be coupled to AE105 thereby providing NOTA-AE105 (NOTA-Asp-Cha-Phe-Ser-Arg-Tyr-Leu-Trp-Ser)). This can be illustrated by the following chemical structure:
In an embodiment, the imaging agent is 68Ga-NOTA-AE105.

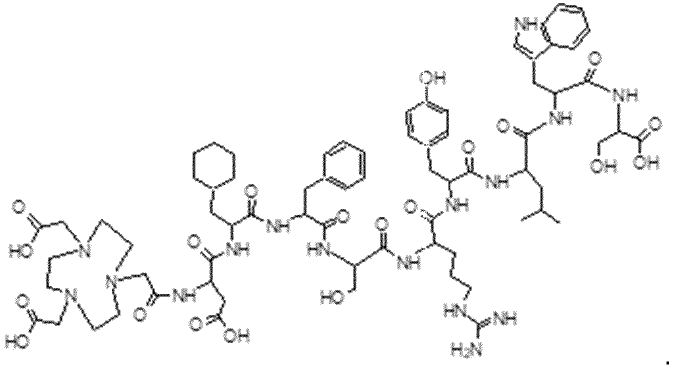

### DOTA

DOTA (also known as tetraxetan) is an organic compound with the formula (CH₂CH₂NCH₂CO₂H)₄. The molecule consists of a central 12-membered tetraaza (i.e., containing four nitrogen atoms) ring. DOTA is used as a complexing agent, especially for lanthanide ions. Its complexes have medical applications as contrast agents and cancer treatments.

Preferred IUPAC name of DOTA 2,2',2",2"'-(1,4,7,10-Tetraazacyclododecane-1,4,7,10-tetrayl)tetraacetic acid Further below DOTA is shown in complex with 68Ga and 64CU coupled to AE105.

### Relapse-free survival (RFS)

The term "relapse-free survival" (RFS) is defined as clinical endpoint defined as time from diagnosis to any relapse of the disease at the locoregional (TN site) and/or distant metastasis (M site) with deaths from other causes recorded as censoring and disease-free survival (DFS), which is defined as RFS, but includes death of any reason as an event.

### Loco-regional control (LRC)

The term "Loco-regional control" (LRC) is defined as time from diagnosis to relapse at the locoregional site with deaths and distant metastasis recorded as censoring.

### Overall survival (OS)

The term "Overall survival" (OS) is defined as time from diagnosis to death of any cause. Follow-up time was calculated from the time of referral to radiotherapy until first relapse, death or until end of follow-up January 1^{st}, 2021.

### Threshold level

In the context of the present invention, the term "threshold level", "reference level" or "cut-off" relates to a standard in relation to a quantity, which other values or characteristics can be compared to.

In one embodiment of the present invention, it is possible to determine a threshold level by investigating the uPAR levels by PET/CT from healthy subjects. By applying different statistical means, such as cut-off finding, multivariate analysis, one or more threshold level can be calculated.

See also example 5, where cut-offs are determined.

Based on these results, a cut-off may be obtained that shows the relationship between the level(s) detected and patients at risk. The cut-off can thereby be used e.g. to determine the uPAR levels, which for instance corresponds to an increased risk of a poor RFS or OS.

### Risk Assessment

The present inventors have successfully developed a new method to predict the prognosis of a head and neck cancer (HNSCC) in a subject, such as RFS and/or OS. To determine whether a patient has an increased risk of a poor prognosis, a cut-off (reference level) must be established. This cut-off may be established by the laboratory, the physician or on a case-by-case basis for each patient.

The cut-off level could be established using a number of methods, including: multivariate statistical tests (such as partial least squares discriminant analysis (PLS-DA), random forest, support vector machine, etc.), percentiles, mean plus or minus standard deviation(s); median value; fold changes.

The multivariate discriminant analysis and other risk assessments can be performed on the free or commercially available computer statistical packages (SAS, SPSS, Matlab, R, etc.) or other statistical software packages or screening software known to those skilled in the art.

As obvious to one skilled in the art, in any of the embodiments discussed above, changing the risk cut-off level could change the results of the discriminant analysis for each subject.

Statistics enables evaluation of the significance of each level. Commonly used statistical tests applied to a data set include t-test, f-test or even more advanced tests and methods of comparing data. Using such a test or method enables the determination of whether two or more samples are significantly different or not. The significance may be determined by the standard statistical methodology known by the person skilled in the art.

The chosen reference level may be changed depending on the mammal/subject for which the test is applied.

Preferably, the subject according to the invention is a human subject.

The chosen reference level may be changed if desired to give a different specificity or sensitivity as known in the art. Sensitivity and specificity are widely used statistics to describe and quantify how good and reliable a biomarker or a diagnostic test is. Sensitivity evaluates how good a biomarker or a diagnostic test is at detecting a disease, while specificity estimates how likely an individual (i.e. control, patient without disease) can be correctly identified as not at risk. Several terms are used along with the description of sensitivity and specificity; true positives (TP), true negatives (TN), false negatives (FN) and false positives (FP). If a disease is proven to be present in a sick patient, the result of the diagnostic test is considered to be TP. If a disease is not present in an individual (i.e. control, patient without disease), and the diagnostic test confirms the absence of disease, the test result is TN. If the diagnostic test indicates the presence of disease in an individual with no such disease, the test result is FP. Finally, if the diagnostic test indicates no presence of disease in a patient with disease, the test result is FN.

### Sensitivity

As used herein the sensitivity refers to the measures of the proportion of actual positives, which are correctly identified as such, i.e. the percentage of subjects having a risk of a poor prognosis above normal who are identified as having a poor prognosis above normal.

Usually the sensitivity of a test can be described as the proportion of true positives of the total number with the target disorder i.e. a risk of poor prognosis above normal. All patients with the target disorder are the sum of (detected) true positives (TP) and (undetected) false negatives (FN).

### Specificity

As used herein the specificity refers to measures of the proportion of negatives which are correctly identified - i.e. the percentage of mammal with a non-increased risk of poor prognosis that are identified as not having a risk of poor prognosis above normal. The ideal diagnostic test is a test that has 100 % specificity, i.e. only detects subjects with a risk of having poor prognosis above normal and therefore no false positive results, and 100% sensitivity, i.e. detects all subjects with a risk of having poor prognosis above normal and therefore no false negative results.

For any test, there is usually a trade-off between each measure. For example, in a manufacturing setting in which one is testing for faults, one may be willing to risk discarding functioning components (low specificity), in order to increase the chance of identifying nearly all faulty components (high sensitivity). This trade-off can be represented graphically using a ROC curve.

The chosen specificity determines the percentage of false positive cases that can be accepted in a given study/population and by a given institution. By decreasing specificity an increase in sensitivity is achieved.

As will be generally understood by those skilled in the art, methods for screening for prognosis are processes of decision making and therefore the chosen specificity and sensitivity depends on what is considered to be the optimal outcome by a given institution/clinical personnel.

### SUVmax

In the present context, the term "SUVmax" refers to the "maximum standardized uptake value" (SUVmax) which is widely used for measuring the uptake of uPAR and FDG by malignant tissue. Increased uptake values reflect increased uptake or binding of the tracers to cancer cells, and can be imaged and quantified using PET.

### SUVmean

In the present context the term "SUVmean", refers to the mean standardized uptake value.

### Imaging agent for use in the prognostication of a head and neck cancer (HNSCC) patient by PET imaging

Improved prognostication of head and neck cancer (HNSCC) patients are important, e.g. for the clinician to be able to select a de-escalated treatment regimen to avoid unnecessary toxicity. Thus, an aspect of the invention relates to a positron-emitting imaging agent for use in the prognostication of a head and neck cancer (HNSCC) patient by PET imaging of the cancer,
wherein said imaging agent comprises a uPAR binding peptide coupled via the chelating agent NOTA to the radionuclide 68Ga; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof.

As outlined in the example section (see example 5), in a multivariate model including uPAR-PET and the routinely used 18F-FDG-PET scan, TNM stage and p16 status, it has surprisingly been found that only uPAR-PET remained significant regarding prognosis expressed as RFS.

Different uPAR binding variants of the uPAR binding peptide (including AE105) are disclosed in WO2014/086364 A1. Thus, in an embodiment, the uPAR binding variant is selected from the group consisting of
- (D-Asp)-([beta]-cyclohexyl-L-alanine)-(Phe)-(D-Ser)-(D-Arg)-(Tyr)-(Leu)-(Trp)-(Ser),
- (Ser)-(Leu)-([beta]-cyclohexyl-L-alanine)-(Phe)-(D-Ser)-(Gln)-(Tyr)(Leu)-(Trp)-(Ser),
- (D-Glu)-([beta]-cyclohexyl-L-alanine)-(Phe)-(D-Ser)-(D-Tyr)-(Tyr)-(Leu)-(Trp)-(Ser),
- (Asp)-([beta]-cyclohexyl-L-alanine)-(Phe)-(D-Ser)-(D-Arg)-(Tyr)-(Leu)-(Trp)-(Ser),
- (Asp)-([beta]-cyclohexyl-L-alanine)-(Phe)-(Ser)-(D-Arg)-(Tyr)-Leu)-(Trp)-(Ser),
- (D-Asp)-([beta]-cyclohexyl-L-alanine)-(Phe)-(Ser)-(D-Arg)-(Tyr)-Leu)-(Trp)-(Ser),
- (D-Thr)-([beta]-cyclohexyl-L-alanine)-(Phe)-(D-Ser)-(D-Arg)-(Tyr)-(Leu)-(Trp)-(Ser),
- (D-Asp)-([beta]-cyclohexyl-L-alanine)-(Phe)-(D-Ser)-(D-Arg)-(Tyr)-(Leu)-([beta]-2-naphthyl-L-alanine)-(Ser),
- (Asp)-([beta]-cyclohexyl-L-alanine)-(Phe)-(D-Ser)-(Arg)-(Tyr)-(Leu)-(Trp)-(Ser),
- (Asp)-([beta]-cyclohexyl-L-alanine)-(Phe)-(D-Ser)-(D-Arg)-(Tyr)-(Leu)([beta]-1-naphthyl-L-alanine)-(Ser),
- (D-Glu)-([beta]-cyclohexyl-L-alanine)-(Phe)-(D-Ser)-(Tyr)-(Tyr)-(Leu)-(Trp)-(Ser),
- (Asp)-([beta]-cyclohexyl-L-alanine)-(Phe)-(D-Ser)-(D-Arg)-(Leu)-(Leu)-(Trp)-(D-His),
- (Asp)-([beta]-cyclohexyl-L-alanine)-(Phe)-(D-Ser)-(D-Arg)-([beta]-cyclohexyl-L-alanine)-(Leu)-(Trp)-(Ile),
- (Asp)-([beta]-cyclohexyl-L-alanine)-(Phe)-(D-Ser)-(D-Arg)-(Tyr)-(Leu)([beta]-1-naphthyl-L-alanine)-(D-His),
- (Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
- (Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
- (Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine), and
- (Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(Ile).

The uPAR binding part of the imaging agent is preferably the one known as AE105. Thus, in an embodiment, peptide is (D-Asp)-([beta]-cyclohexyl-L-alanine)-(Phe)-(D-Ser)-(D-Arg)-(Tyr)-(Leu)-(Trp)-(Ser).

In another embodiment, the C-terminal is a carboxylic acid or an amide.

In yet an embodiment, the imaging agent has the formula

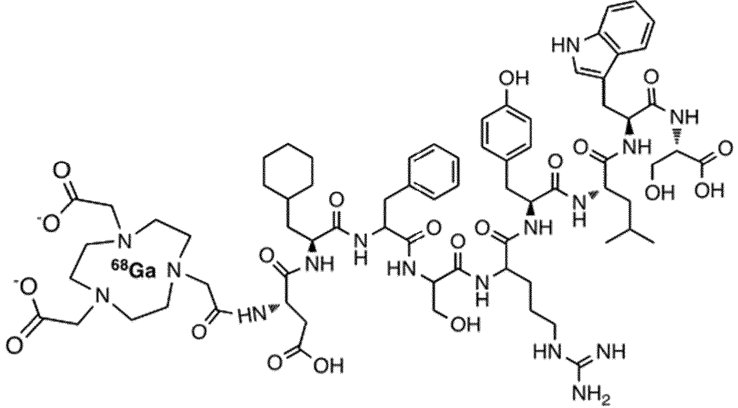

To further improve localization of the cancer, the PET scan may be combined with other scanning types. Thus, in an embodiment, prognostication comprises PET/CT scanning and/or PET/MR scanning. In the example section, PET/CT has been used.

The prognostication may be further defined. Thus, in an embodiment, the prognosis is prognostication of relapse-free survival (RFS) and/or overall survival (OS). In the example section, RFS and OS have been assessed.

In a preferred embodiment, the prognostication is relapse-free survival (RFS). Again, as shown e.g. in example 5, in a multivariate model including uPAR-PET and the routinely used 18F-FDG-PET scan, TNM stage and p16 status, it has surprisingly been found that only uPAR-PET remained significant regarding prognosis expressed as RFS.

Different amount (MBq) of the imaging may be used. Also the time before scanning after administration of the imaging agent may vary. Thus, in an embodiment, the imaging agent is to be administered in a dose of 20-500 MBq, such as 100-500 MBq followed by PET scanning 10 min to 24 hours after the imaging agent has been administered, and quantification through SUVmax and/or SUVmean.

In another embodiment, the imaging agent is to be administered in a dose of 20-400 MBq, such as 50-400 MBq, such as 70-300 MBq, such as 100-300 MBq or such as 100-300 MBq, preferably such as 150-250 MBq, and more preferably 170-230 MBq. Thus in a preferred embodiment, the imaging agent is to be administered in a dose of 70-300 MBq, preferably such as 150-250 MBq. In the example section, around 200 MBq has been used and PET imaging was performed with a Siemens Biograph mCT 64 slice. However, other equipment may be more sensitive and therefore allow for lower amount (MBq) of the imaging agent.

In an embodiment, a sufficient amount of imaging aging is administered to allow for PET imaging with enough radioactivity dose for imaging.

68Ga has a half-life of around 1 hour (68 min) making 10 minutes to 5 hours, preferably 20 minutes to 3 hours realistic time before PET scanning. 64Cu has a half-life of around 12.7 hours making an interval of 20 minutes to 24 hours before PET scanning realistic.

Thus, in a related embodiment, the PET scanning follows 20 minutes to 10 hours after the imaging agent has been administered, such as 20 minutes to 5 hours, such as 30 minutes to 3 hours after the imaging agent has been administered.

In yet another embodiment, the imaging agent according to the invention is in a pharmaceutical composition comprising the imaging agent, together with one or more pharmaceutical acceptable adjuvants, excipients and/or diluents.

To be able to make a prognostication a threshold level (cut-off/reference level) may be included. Thus, in an embodiment, for the imaging agent for use according to the invention,
∘ a SUVmax and/or SUVmean quantification level above a threshold level is indicative of a poor Relapse-free survival (RFS) prognosis and/or poor overall survival (OS) prognosis; and
∘ a SUVmax and/or SUVmean quantification level equal to or below a threshold level is indicative of good Relapse-free survival (RFS) prognosis and/or good overall survival (OS) prognosis.

In example 5, specific optimal threshold levels (cut-offs) has been calculated (see also figures 3A-D).

As also outlined in example 5, the imaging agent according to the invention is particular efficient in the prognosis of Relapse-free survival (RFS). Thus, in an embodiment,
- a SUVmax and/or SUVmean quantification level above a threshold level is indicative of a poor Relapse-free survival (RFS) prognosis; and
- a SUVmax and/or SUVmean quantification level equal to or below a threshold level is indicative of good Relapse-free survival (RFS) prognosis.

In yet an embodiment, said SUVmax and/or SUVmean threshold level is in the range 1-4, such as in the range 2-4, preferably in the range 2-3, such as 2.4-2.8. SUVmax and/or SUVmean threshold levels have been calculated in example 5.

In yet an embodiment, said threshold level is determined by using a cut-off finding method to obtain a split in a Kaplan-Meier plot (log-rank test) and the corresponding hazard ratios (HRs).

In yet another embodiment, said human patient has been referred to curatively intended radiotherapy.

In a further embodiment, the HNSCC is located in the pharynx, larynx or oral cavity.

In an embodiment said prognosis further encompasses one or more of 18F-FDG-PET scan, TNM stage and p16 status. However, it is noted that it has surprisingly been found that only uPAR-PET remained significant regarding prognosis expressed as RFS.

In a preferred aspect the invention relates to a positron-emitting imaging agent for use in the prognostication of relapse-free survival (RFS) and/or overall survival (OS) of a head and neck squamous cell carcinoma (HNSCC) in a human patient by PET imaging of the cancer,
wherein said imaging agent comprises a uPAR binding peptide coupled via the chelating agent NOTA to the radionuclide 68Ga; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof;
wherein the uPAR binding variant is selected from the group consisting of
   - (D-Asp)-([beta]-cyclohexyl-L-alanine)-(Phe)-(D-Ser)-(D-Arg)-(Tyr)-(Leu)-(Trp)-(Ser),
   - (Ser)-(Leu)-([beta]-cyclohexyl-L-alanine)-(Phe)-(D-Ser)-(Gln)-(Tyr)(Leu)-(Trp)-(Ser),
   - (D-Glu)-([beta]-cyclohexyl-L-alanine)-(Phe)-(D-Ser)-(D-Tyr)-(Tyr)-(Leu)-(Trp)-(Ser),
   - (Asp)-([beta]-cyclohexyl-L-alanine)-(Phe)-(D-Ser)-(D-Arg)-(Tyr)-(Leu)-(Trp)-(Ser),
   - (Asp)-([beta]-cyclohexyl-L-alanine)-(Phe)-(Ser)-(D-Arg)-(Tyr)-Leu)-(Trp)-(Ser),
   - (D-Asp)-([beta]-cyclohexyl-L-alanine)-(Phe)-(Ser)-(D-Arg)-(Tyr)-Leu)-(Trp)-(Ser),
   - (D-Thr)-([beta]-cyclohexyl-L-alanine)-(Phe)-(D-Ser)-(D-Arg)-(Tyr)-(Leu)-(Trp)-(Ser),
   - (D-Asp)-([beta]-cyclohexyl-L-alanine)-(Phe)-(D-Ser)-(D-Arg)-(Tyr)-(Leu)-([beta]-2-naphthyl-L-alanine)-(Ser),
   - (Asp)-([beta]-cyclohexyl-L-alanine)-(Phe)-(D-Ser)-(Arg)-(Tyr)-(Leu)-(Trp)-(Ser),
   - (Asp)-([beta]-cyclohexyl-L-alanine)-(Phe)-(D-Ser)-(D-Arg)-(Tyr)-(Leu)([beta]-1-naphthyl-L-alanine)-(Ser),
   - (D-Glu)-([beta]-cyclohexyl-L-alanine)-(Phe)-(D-Ser)-(Tyr)-(Tyr)-(Leu)-(Trp)-(Ser),
   - (Asp)-([beta]-cyclohexyl-L-alanine)-(Phe)-(D-Ser)-(D-Arg)-(Leu)-(Leu)-(Trp)-(D-His),
   - (Asp)-([beta]-cyclohexyl-L-alanine)-(Phe)-(D-Ser)-(D-Arg)-([beta]-cyclohexyl-L-alanine)-(Leu)-(Trp)-(Ile),
   - (Asp)-([beta]-cyclohexyl-L-alanine)-(Phe)-(D-Ser)-(D-Arg)-(Tyr)-(Leu)([beta]-1-naphthyl-L-alanine)-(D-His),
   - (Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
   - (Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
   - (Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine), and
   - (Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(Ile);

   - wherein a SUVmax and/or SUVmean quantification level above a threshold level is indicative of a poor Relapse-free survival (RFS) prognosis and/or poor overall survival (OS) prognosis; and
   - wherein a SUVmax and/or SUVmean quantification level equal to or below a threshold level is indicative of good Relapse-free survival (RFS) prognosis and/or good overall survival (OS) prognosis.

### Method of in vivo imaging by PET imaging, for assessing the prognosis of a head and neck cancer (HNSCC) in a patient

Another aspect of the invention relates to a method of in vivo imaging by PET imaging, for assessing the prognosis of a head and neck cancer (HNSCC) in a patient, said method comprising:
a) provision of a subject to which a imaging agent according to the invention has been previously administered;
b) detecting by in vivo PET imaging the radioactive emissions from the radioisotope of the administered imaging agent of step a);
c) generating an image representative of the location and/or amount of said radioactive emissions;
d) determining the distribution and extent of uPAR expression in said subject, wherein said expression is correlated with said signals emitted by said in vivo imaging agent; and
e) comparing the determined distribution and extent of uPAR expression to a threshold level;
   ▪ wherein a SUVmax and/or SUVmean quantification level above a threshold level is indicative of a poor Relapse-free survival (RFS) prognosis and/or poor overall survival (OS) prognosis; and
   ▪ wherein a SUVmax and/or SUVmean quantification level equal to or below a threshold level is indicative of good Relapse-free survival (RFS) prognosis and/or good overall survival (OS) prognosis.

The prognostication according to the invention, may be used by the clinician to select a treatment protocol for the patient, such as a curatively intended radiotherapy. Thus, in an embodiment, if a good Relapse-free survival (RFS) prognosis is indicated, said subject may be scheduled for a de-escalated treatment regimen to avoid unnecessary toxicity and/or for a risk stratified follow-up schedule.In an embodiment, said treatment is curatively intended radiotherapy.

In the present context, a de-escalated treatment protocol is a protocol, which is considered less toxic/harmful to the patient compared to a patient who has received a poor Relapse-free survival (RFS) prognosis.

In an embodiment, said de-escalated treatment being abstaining from or reducing radiotherapy.

### Use of a positron-emitting imaging agent in the prognosis of head and neck cancer (HNSCC) patients by in vivo PET imaging

A further aspect of the invention relates to the use of a positron-emitting imaging agent according to the invention in the prognosis of head and neck cancer (HNSCC) patients by in vivo PET imaging of uPAR expressing tumors,
wherein said imaging agent comprises a uPAR binding peptide coupled via the chelating agent NOTA to the radionuclide 68Ga;
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof.

In an alternative part of this aspect, said imaging agent comprises a uPAR binding peptide coupled via the chelating agent NOTA or DOTA to the radionuclide 68Ga or 64Cu.

Again, in an embodiment, the uPAR binding variant thereof is selected from the group consisting of
- (D-Asp)-([beta]-cyclohexyl-L-alanine)-(Phe)-(D-Ser)-(D-Arg)-(Tyr)-(Leu)-(Trp)-(Ser),
- (Ser)-(Leu)-([beta]-cyclohexyl-L-alanine)-(Phe)-(D-Ser)-(Gln)-(Tyr)(Leu)-(Trp)-(Ser),
- (D-Glu)-([beta]-cyclohexyl-L-alanine)-(Phe)-(D-Ser)-(D-Tyr)-(Tyr)-(Leu)-(Trp)-(Ser),
- (Asp)-([beta]-cyclohexyl-L-alanine)-(Phe)-(D-Ser)-(D-Arg)-(Tyr)-(Leu)-(Trp)-(Ser),
- (Asp)-([beta]-cyclohexyl-L-alanine)-(Phe)-(Ser)-(D-Arg)-(Tyr)-Leu)-(Trp)-(Ser),
- (D-Asp)-([beta]-cyclohexyl-L-alanine)-(Phe)-(Ser)-(D-Arg)-(Tyr)-Leu)-(Trp)-(Ser),
- (D-Thr)-([beta]-cyclohexyl-L-alanine)-(Phe)-(D-Ser)-(D-Arg)-(Tyr)-(Leu)-(Trp)-(Ser),
- (D-Asp)-([beta]-cyclohexyl-L-alanine)-(Phe)-(D-Ser)-(D-Arg)-(Tyr)-(Leu)-([beta]-2-naphthyl-L-alanine)-(Ser),
- (Asp)-([beta]-cyclohexyl-L-alanine)-(Phe)-(D-Ser)-(Arg)-(Tyr)-(Leu)-(Trp)-(Ser),
- (Asp)-([beta]-cyclohexyl-L-alanine)-(Phe)-(D-Ser)-(D-Arg)-(Tyr)-(Leu)([beta]-1-naphthyl-L-alanine)-(Ser),
- (D-Glu)-([beta]-cyclohexyl-L-alanine)-(Phe)-(D-Ser)-(Tyr)-(Tyr)-(Leu)-(Trp)-(Ser),
- (Asp)-([beta]-cyclohexyl-L-alanine)-(Phe)-(D-Ser)-(D-Arg)-(Leu)-(Leu)-(Trp)-(D-His),
- (Asp)-([beta]-cyclohexyl-L-alanine)-(Phe)-(D-Ser)-(D-Arg)-([beta]-cyclohexyl-L-alanine)-(Leu)-(Trp)-(Ile),
- (Asp)-([beta]-cyclohexyl-L-alanine)-(Phe)-(D-Ser)-(D-Arg)-(Tyr)-(Leu)([beta]-1-naphthyl-L-alanine)-(D-His),
- (Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
- (Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
- (Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine), and
- (Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(Ile).

### Additional aspects of the invention

An additional aspect of the invention relates to a positron-emitting imaging agent for use in the prognostication of a head and neck cancer (HNSCC) patient by PET imaging of the cancer,
wherein said imaging agent comprises a uPAR binding peptide coupled via the chelating agent NOTA or DOTA to the radionuclide 68Ga or 64Cu; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof.

Again, in an embodiment, uPAR binding variant thereof is selected from the group consisting of
- (D-Asp)-([beta]-cyclohexyl-L-alanine)-(Phe)-(D-Ser)-(D-Arg)-(Tyr)-(Leu)-(Trp)-(Ser),
- (Ser)-(Leu)-([beta]-cyclohexyl-L-alanine)-(Phe)-(D-Ser)-(Gln)-(Tyr)(Leu)-(Trp)-(Ser),
- (D-Glu)-([beta]-cyclohexyl-L-alanine)-(Phe)-(D-Ser)-(D-Tyr)-(Tyr)-(Leu)-(Trp)-(Ser),
- (Asp)-([beta]-cyclohexyl-L-alanine)-(Phe)-(D-Ser)-(D-Arg)-(Tyr)-(Leu)-(Trp)-(Ser),
- (Asp)-([beta]-cyclohexyl-L-alanine)-(Phe)-(Ser)-(D-Arg)-(Tyr)-Leu)-(Trp)-(Ser),
- (D-Asp)-([beta]-cyclohexyl-L-alanine)-(Phe)-(Ser)-(D-Arg)-(Tyr)-Leu)-(Trp)-(Ser),
- (D-Thr)-([beta]-cyclohexyl-L-alanine)-(Phe)-(D-Ser)-(D-Arg)-(Tyr)-(Leu)-(Trp)-(Ser),
- (D-Asp)-([beta]-cyclohexyl-L-alanine)-(Phe)-(D-Ser)-(D-Arg)-(Tyr)-(Leu)-([beta]-2-naphthyl-L-alanine)-(Ser),
- (Asp)-([beta]-cyclohexyl-L-alanine)-(Phe)-(D-Ser)-(Arg)-(Tyr)-(Leu)-(Trp)-(Ser),
- (Asp)-([beta]-cyclohexyl-L-alanine)-(Phe)-(D-Ser)-(D-Arg)-(Tyr)-(Leu)([beta]-1-naphthyl-L-alanine)-(Ser),
- (D-Glu)-([beta]-cyclohexyl-L-alanine)-(Phe)-(D-Ser)-(Tyr)-(Tyr)-(Leu)-(Trp)-(Ser),
- (Asp)-([beta]-cyclohexyl-L-alanine)-(Phe)-(D-Ser)-(D-Arg)-(Leu)-(Leu)-(Trp)-(D-His),
- (Asp)-([beta]-cyclohexyl-L-alanine)-(Phe)-(D-Ser)-(D-Arg)-([beta]-cyclohexyl-L-alanine)-(Leu)-(Trp)-(Ile),
- (Asp)-([beta]-cyclohexyl-L-alanine)-(Phe)-(D-Ser)-(D-Arg)-(Tyr)-(Leu)([beta]-1-naphthyl-L-alanine)-(D-His),
- (Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
- (Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
- (Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine), and
- (Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(Ile).

In an embodiment, the imaging agent comprises 68Ga-NOTA and the uPAR binding peptide as outlined for the invention.

In an embodiment, the imaging agent comprises 68Ga-DOTA and the uPAR binding peptide as outlined for the invention. In an embodiment, the imaging agent has the formula:

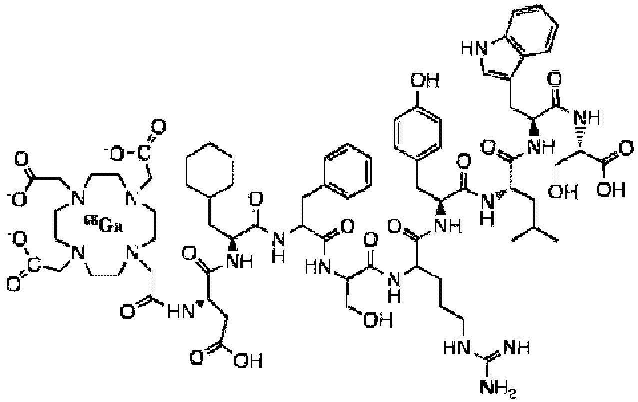

In an embodiment, the imaging agent comprises 64Cu-NOTA and the uPAR binding peptide as outlined for the invention. In an embodiment, the imaging agent has the formula:

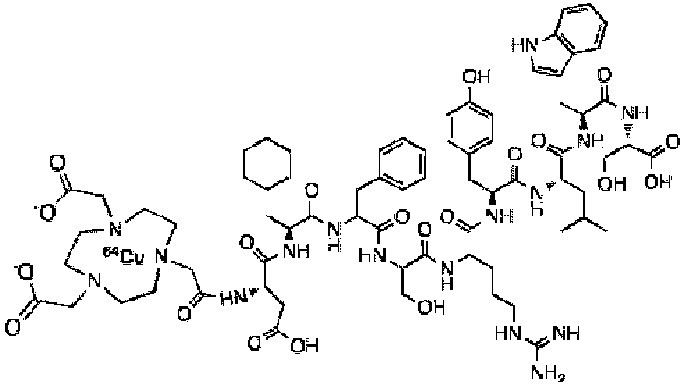

In an embodiment, the imaging agent comprises 64Cu-DOTA and the uPAR binding peptide as outlined for the invention. In an embodiment, the imaging agent has the formula:

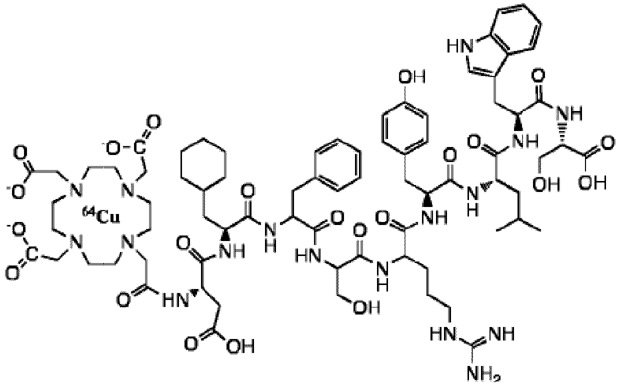

The different variants of the radionuclide-chelator-uPAR binding peptide (including AE105) are also disclosed in WO2014/086364 A1**.**

The two uPAR-PET tracers 64Cu-DOTA-AE105 and 68Ga-NOTA-AE105 share the same binding moiety, the peptide AE105. In addition, they have both been tested in both humans and animals (e.g. 15, 17 and WO2014/086364 A1) and have demonstrated similar uptake in breast, bladder and prostate cancer. Accordingly, without being bound by theory, it is believed the two PET tracers will work in a similar manner also for HNSCC patients.

The invention will now be described in further details in the following non-limiting examples.

### Examples

### Example 1 - Materials and methods

### Patient population

Inclusion criteria were patients with a diagnosis of biopsy-verified cancer of the pharynx, larynx or oral cavity, referred to curatively intended radiotherapy, who understood the given information, was able to give informed consent and age above 18 years.

Exclusion criteria were pregnancy, lactation/breast feeding, age above 85 years, obesity (bodyweight above 140 kg), small cancers of the larynx (1A,1B), allergy to 68Ga-NOTA-AE105, metastasis on FDG-PET/CT, other previously known cancers and claustrophobia. Eligible patients were included after giving informed consent. Diagnosis of HNSCC and p16 status was verified histologically prior to inclusion. Information on smoking, alcohol, clinical examination, treatment plan, laboratory and histological results, medical history and follow-up examinations were collected from patient records. Disease stage was coded according to Union for International Cancer Control (UICC) 8^{th} edition.

### 68Ga-uPAR-PET/CT acquisition

According to current national guidelines on treatment of HNSCC, radiotherapy is to be initiated within 11 days of the treatment decision and 18F-FDG-PET/CT and 68Ga-uPAR-PET/CT were performed within this period, both as a part of the prospective study. In order to minimize the risk of osteonecrosis following radiotherapy, patients underwent a dental examination and in the case of teeth extraction, initiation of radiotherapy was postponed until two weeks after the procedure. In this case, 18F-FDG-PET/CT and 68Ga-uPAR-PET/CT was scheduled before or at least 4 days after the procedure.

All patients were injected intravenously with approximately 200 MBq (median 191, range 158-209 MBq) of 68Ga-NOTA-AE105 followed by sequential whole-body PET/CT scanning 20 min post-injection. Whole-body 68Ga-NOTA-AE105 PET and diagnostic CT with contrast (skull base to proximal thigh) was performed simultaneously using an integrated whole-body PET/CT (Siemens Biograph mCT 64 slice, Siemens, Erlangen, Germany). Synthesis of the ligand was performed as previously described (15).

Patients were immobilized in the supine position on a flat scanner couch with arms placed in standard anatomical position and no fixating facial mask was applied. The CT scan was performed with 120kV, 170 MAS, pitch 0.8. The PET data was reconstructed with an iterative reconstruction method using time of flight, point spread function and attenuation corrections with 2 iterations, 21 subsets and a 2 mm Gaussian filter.

### Image Analysis

Image data from the 68Ga-uPAR-PET/CT and 18F-FDG-PET/CT was analyzed by two certified specialists in nuclear medicine. The readers were blinded to the volumes of interest (VOIs), results of the other reader and patient information. Volumes of interest were visually contoured on the 68Ga-uPAR-PET/CT corresponding to the localization of the primary tumor on the 18F-FDG-PET/CT. Uptake of the 68Ga-uPAR ligand and 18F-FDG in the volumes of interest were parameterized as SUVmax on the 68Ga-uPAR-PET/CT and 18F-FDG-PET/CT and documented for both tracers before obtaining information of recurrences and survival.

If a patient had two synchronous primary HNSCCs, the tumor with the highest SUVmax was included in the statistical analysis. The mean value of the SUVmax obtained by the two independent readers was included in the statistical analysis.

### Treatment and Follow-up

All patients received intensity-modulated radiotherapy (IMRT) with or without concomitant chemotherapy according to national guidelines (18). All patients received a prescribed dose of 66 to 68 Gy in 33 to 34 fractions, 6 fractions per week and one patient received proton radiation. Patients with advanced disease, if assessed fit, received concomitant cisplatin administered weekly (40 mg/m2); all patients with normal liver and renal function tests and no neurologic symptoms received a hypoxic radiosensitizer (nimorazole) daily (1200 mg/m2). According to national guidelines, all HNSCC patients attended a 5-year follow-up program. All patients attended the follow-up program throughout the study period and concurrent diseases, visits to other departments and deceased individuals were followed through the Danish personal identity number.

### Statistical Analysis

A sample size of 104 patients was calculated as needed for the study based on ability to detect a HR of 2.5 with a power of 70% (beta: 30%) and a level of significance (alpha) of 5% and a follow up of 2 years. However, due to the slowdown in performing clinical studies caused by the COVID-19 pandemic the study was delayed, but with a longer follow-up the needed number of events was reached.

Clinical endpoints were relapse-free survival (RFS) defined as time from diagnosis to any relapse of the disease at the locoregional (TN site) and/or distant metastasis (M site) with deaths from other causes recorded as censoring and disease-free survival (DFS), which is defined as RFS, but includes death of any reason as an event. Loco-regional control (LRC) was defined as time from diagnosis to relapse at the locoregional site with deaths and distant metastasis recorded as censoring. Overall survival (OS) was defined as time from diagnosis to death of any cause. Follow-up time was calculated from the time of referral to radiotherapy until first relapse, death or until end of follow-up January 1^{st}, 2021.

Determination of the optimal cut-off (threshold level) in discrimination between favorable and poor prognosis was performed with Cut-off finder, an R-package developed by Budczies et al.(19). Associations between biomarker expression beneath or above cut-off and survival outcomes were visualized in Kaplan-Meier plots using the log-rank test to assess significance of differences. Hazard ratios were estimated in univariable and multivariable Cox proportional hazards model in which the PET parameters were included as binarized parameters according to the defined cut-offs for RFS and OS.

The number of events included in the survival analysis were: 17 events in RFS and 16 events in OS analysis. Based on the number of events, four predictors were the maximal number of explanatory variables that could reasonably be included in the final multivariable Cox model. In addition to the aim of testing the prognostic value of 68Ga-uPAR SUVmax and compare it to 18F-FDG SUVmax, TNM stage and p16 status (p16-positive OPSCC versus all other tumors) were also included in the multivariable analysis, since they are the most important non-imaging prognostic factors in HNSCC (9). Model performance was estimated using Harrell's Concordance Index (C-index).

Interrater reliability of SUV-measurement was estimated using intraclass correlation coefficient (ICC).

A p-value of < 0.05 was considered statistically significant. Statistical analyses were performed using IBM SPSS Statistics v. 22 (IBM Corp. Armonk, NY) and R (http://www.Rproject.org).

### Example 2 - Patients

A total of fifty-seven patients recently diagnosed with HNSCC in the pharynx, larynx or oral cavity and referred to curatively intended radiotherapy at Rigshospitalet and Naestved Hospital, Denmark, were consecutively included in the current prospective phase II study from December 2017 - November 2019, Figure 1. None of the patients experienced reactions or adverse events related to the administration of 68Ga-NOTA-AE105. One patient terminated the 68Ga-NOTA-AE105-(uPAR)-PET/CT scan due to claustrophobia and two patients were diagnosed with unknown primary tumor (UPT) of the head and neck after a lymph node biopsy and were excluded from the statistical analysis. Patient characteristics are shown in Table 1. More than half the patients (59.2 %) presented with early stage disease (Stage 1-2) and 38.9 % had no primary regional nodal disease. Moreover, 61.1 % were located in the oropharynx of which 78.7 % were p16 positive. Median follow-up was 33.8 months (range 2.30-47.2).

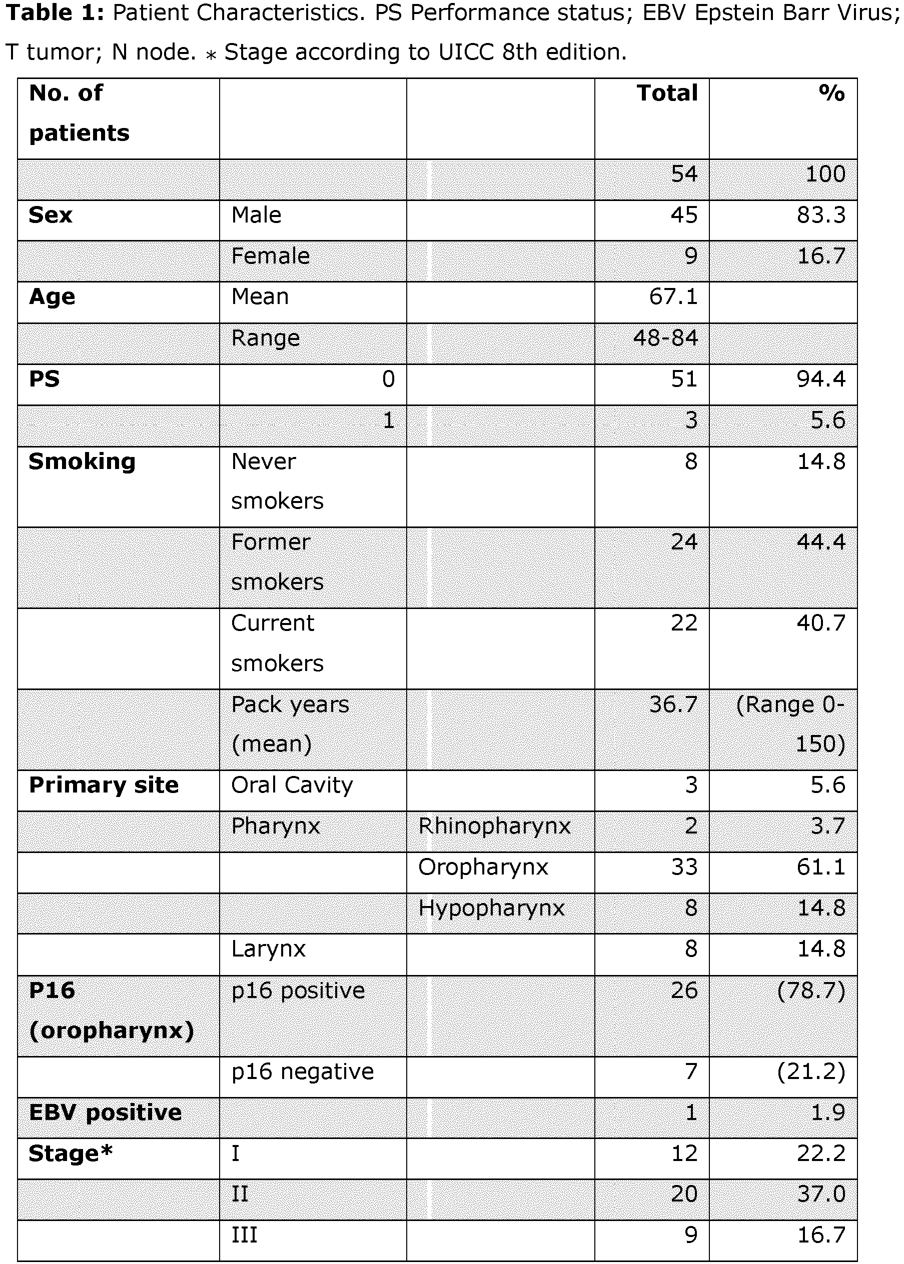

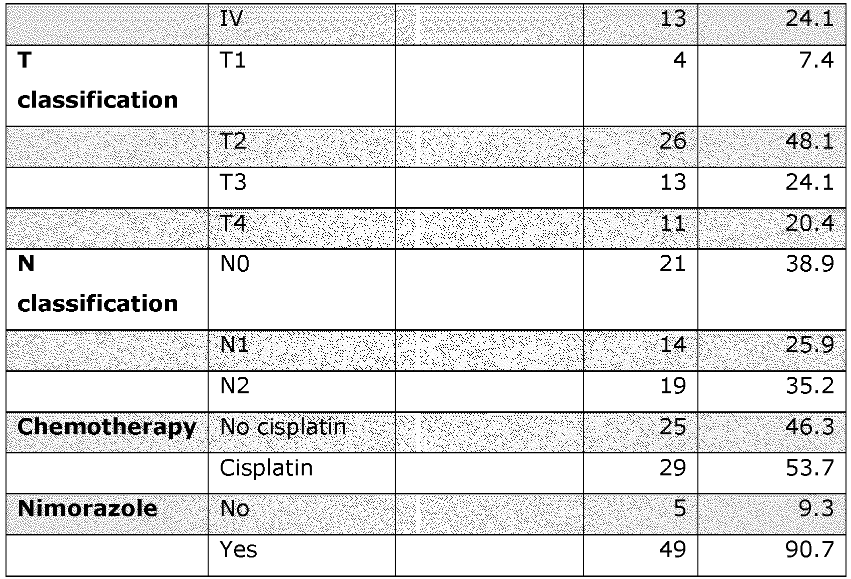

### Example 3 - Clinical Follow-up

Histological verification of loco-regional recurrences was obtained in 15/16 patients serving as reference for the study outcome. One patient did not have a histological verification of the loco-regional recurrence but had active tumor at the primary site on 18F-FDG-PET/CT and histologically verified lung metastases. Biological material from biopsy or surgery was available from all (3) patients with suspected distant metastasis. Consequently, we did not experience missing data regarding recurrences and the two patients with unknown primary tumors (UPT) were excluded due to missing data from the primary tumor. No patients were lost to follow-up and clinicopathological information was collected before inclusion.

Seventeen patients (31.5 %) were diagnosed with a recurrence, 7 (13.0 %) at the primary site (T site), 5 (9.3 %) at the primary site and lymph nodes (TN site), 2 (3.7 %) in the lymph nodes (N site), and 3 (5.6 %) were diagnosed with distant metastases in the lungs (M site). Two of the patients were classified as residual tumor at the 2 months follow-up. Ten of the 17 recurrences (58.8 %) were p16 negative, while seven (41.2 %) were p16 positive tumors. 30 % (3/10) of the loco-regional recurrences were p16 positive and all patients (3/3) with distant metastases were confirmed p16 positive. All 17 patients who experienced a relapse completed all fractions of the primary radiotherapy.

During follow-up 16 patients (29.6 %) died; 8 (14.8 %) due to HNSCC and 8 (14.8 %) due to non-HNSCC causes; 1 (1.9 %) died due to sepsis 1 month after treatment, 1 died due to exacerbation of Chronic Obstructive Pulmonary Disease (COPD), 1 due to lung embolism (diagnosed and successfully operated for a recurrence prior to his death), 1 discontinued his routine treatment of HIV and died due to infection, 1 died of rectal cancer, 2 of lung cancer, and 1 died of unknown causes, but without any sign of recurrence at the follow-up 2 months prior to his death. None of the non-cancer deaths had signs of recurrence at the previous follow-up. The patient who died of sepsis 1 month after treatment before the first routine follow-up was included in the statistical analyses as not having an event. Imaging performed in the acute phase in the case of sepsis and exacerbation of COPD had no sign of recurrence. 4 out of 6 patients who died due to HNSCC were p16 negative (66.7 %), while 2 (33.3 %) were p16 positive.

### Example 4 - 68Ga-uPAR- and 18F-FDG-uptake

The median SUVmax of the primary tumors were 2.98 (range 1.94-5.24) for 68Ga-uPAR-uptake and 15.7 (range 4.24-45.5) for 18F-FDG-uptake (Figure 2). The median time interval between the 68Ga-uPAR- and 18F-FDG-PET/CT was 2.4 days (range 1-4).

### Example 5 - Cut-off Points and Kaplan-Meier Curves

### Aim of study

To determine cut-off Points and Kaplan-Meier Curves.

### Results

The optimal cut-off points (threshold levels), determined as the point with the most significant split in the Kaplan-Meier plot (log-rank test) and the corresponding hazard ratios (HRs) including 95% confidence intervals, were calculated (19).

For 68Ga-uPAR the cut-points were 2.63 for RFS and 2.66 for OS, separating the patients in a group of 41 (75.6%) above cut-off and 13 (24.1%) below cut-off in RFS analysis and a group of 40 (74.1%) above and 14 (25.9%) below cut-off in OS analysis.

For 18F-FDG-PET, the optimized cut-points were 22.7 for RFS and 22.9 for OS, separating a group of 42 (77.8%) patients below and 12 (22.2%) patients above cut-off in RFS analysis, and a group of 43 (79.6%) patients below and 11 (20.4%) patients above cut-off in OS analysis.

### Conclusion

Kaplan-Meier curves combined with log-rank analysis for differences, showed a significant association between:
a) Poor Relapse-free survival (RFS) (log-rank p=0.012) and 68Ga-uPAR SUVmax above cut-off (Figure 3A); and
b) Poor Overall survival (OS) (log-rank p=0.02) and 68Ga-uPAR SUVmax above cut-off (Figure 3B).

Similarly, Kaplan-Meier curves combined with log-rank analysis for differences, showed a significant association between:
a) Reduced Relapse-free survival (RFS) (p=0.012) and 18F-FDG SUVmax above cut-off (Figure 3C); and
b) Poor Overall survival (OS) (p <0.001) and 18F-FDG SUVmax above cut-off (Figure 3D).

Importantly, 68Ga-uPAR SUVmax below the identified cut-off was a surprisingly strong predictor for Relapse free survival (RFS) and overall survival when compared to 18F-FDG (see figure 3). More surprisingly (and importantly) 68Ga-uPAR SUVmax below the identified cut-off was a very strong predictor for Relapse free survival (RFS) compared to 18F-FDG (compare figure 3A and 3C).

Thus, low 68Ga-uPAR may allow the clinician to decide for a de-escalating treatment regime since 68Ga-uPAR is a strong prognostic marker for RFS.

In sum, in the multivariate model including uPAR-PET and the routinely used 18F-FDG-PET scan, TNM stage and p16 status, it has surprisingly been found that only uPAR-PET remained significant regarding prognosis expressed as RFS.

### Example 6 - Survival Analysis

### Aim of study

To perform a survival analysis.

### Results

Univariate and multivariate analysis using Cox proportional hazards model are summarized in Table 2 and Table 3 for RFS and OS respectively.

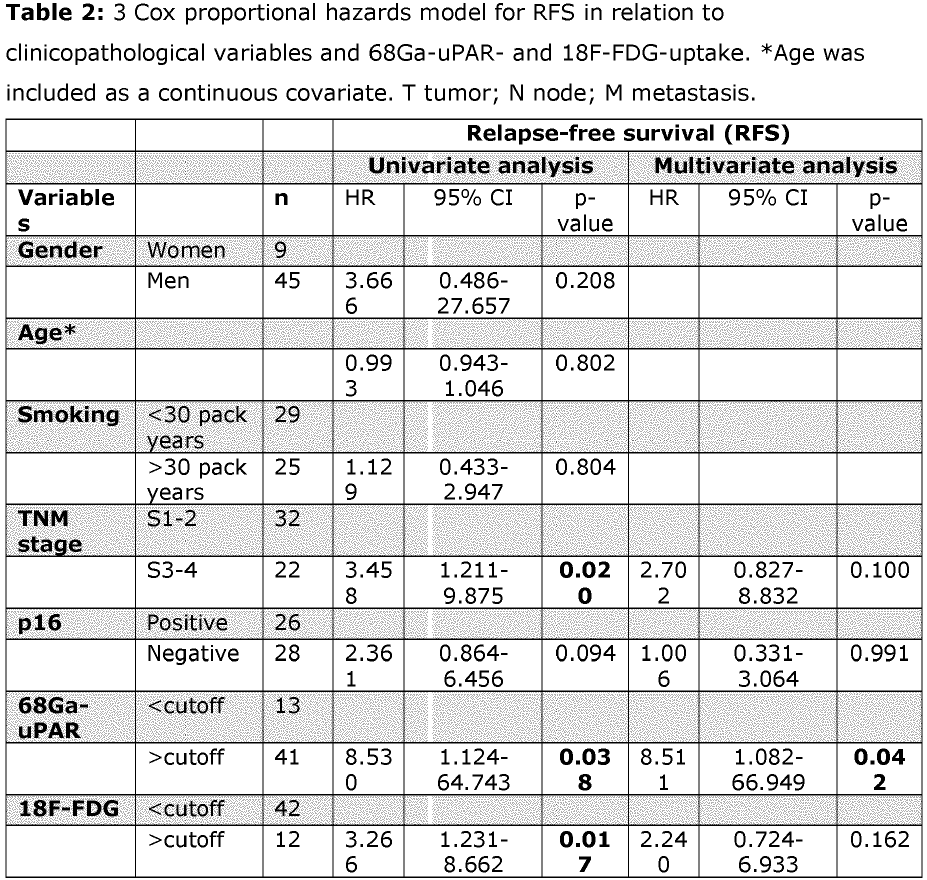

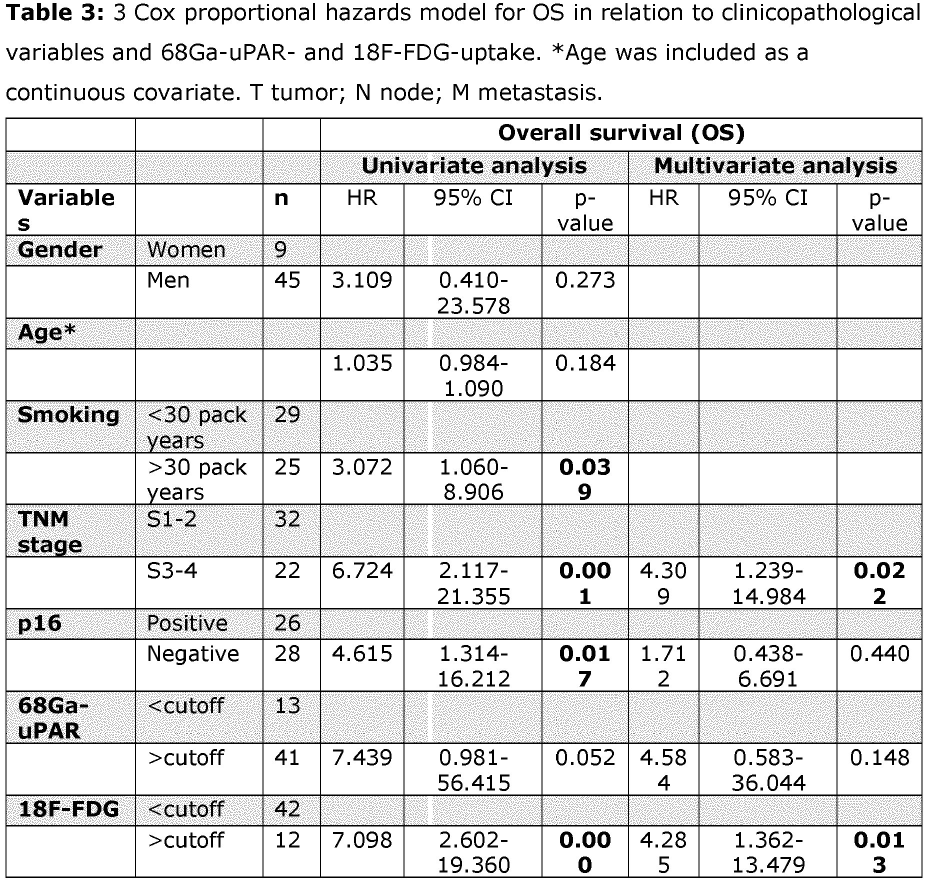

In univariate analysis high uptake of 68Ga-uPAR (above cut-off) in the primary tumor was significantly associated with reduced RFS (HR=8.53 (95% confidence interval (CI) 1.12-64.7), p=0.038) and borderline significant associated with OS (HR=7.44 (95% CI 0.981-56.44), p=0.052). High uptake of 18F-FDG was significantly associated with reduced RFS and OS (HR=3.27 (95% CI 1.237-8.66), p=0.017) and (HR=7.10(95% CI 2.60-19.4), p<0.001). High TNM stage (S3-4) was significantly associated with both RFS (HR=3.46 (95% CI 1.216-9.88), p=0.020) and OS (HR=6.72 (95% CI 2.12-21.4), p=0.001).

In multivariable analysis, including 68Ga-uPAR SUVmax, 18F-FDG SUVmax, TNM stage and p16, only 68Ga-uPAR SUVmax remained significantly associated with RFS (HR 8.50 (95%CI 1.11-65.3), p=0.040), but not with respect to OS (HR = 4.58 (95% CI 0.583-36.0), p=0.148). For OS, high 18F-FDG SUVmax (HR=4.986 (95% CI 1.658-14.990), p=0.004) and TNM stage (HR=3.856 (95% CI 1.114-13.343), p=0.033) remained significantly associated. In DFS analysis, the results reflected the fact that DFS is a combination of RFS and OS events (data not shown). We did not have statistical power to conclude on LRC due to too few events, but the results showed the same trend as RFS.

In post-hoc analysis, inclusion of 68Ga-uPAR SUVmax in the multivariate cox-model improved the predictive performance in RFS analysis (C-index: 0.74 to 0.78) and for 18F-FDG (C-index: 0.76 to 0.78). In OS analysis inclusion of 68Ga-uPAR SUVmax improved the predictive performance (C-index: 0.81 to 0.84) and for 18F-FDG (C-index: 0.80 to 0.84). The C-index for a model only including TNM stage and p16 was 0.70 for RFS and 0.77 for OS.

### Example 7 - 68Ga-uPAR and 18F-FDG Concordance

### Aim of study

To determine 68Ga-uPAR and 18F-FDG concordance.

### Results

In post-hoc analysis, combining 68Ga-uPAR-PET and 18F-FDG into groups of 1) both scans low, 2) one scan high/one scan low and 3) both scans high according to the established cut-offs, demonstrated a concordance rate near 40% for RFS and OS and a discordance rate near 60% for RFS and OS.

The distribution of the groups is shown in table 4 and Kaplan-Meier curves in figures 4A-B.

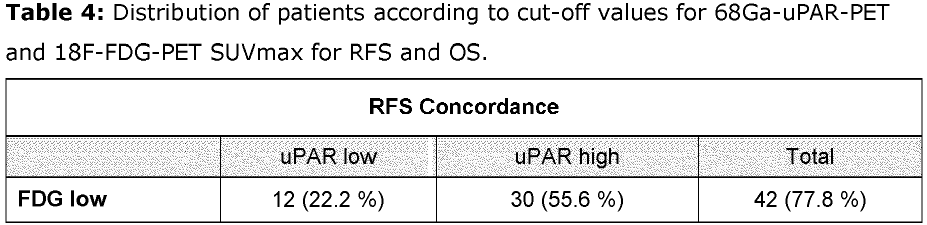

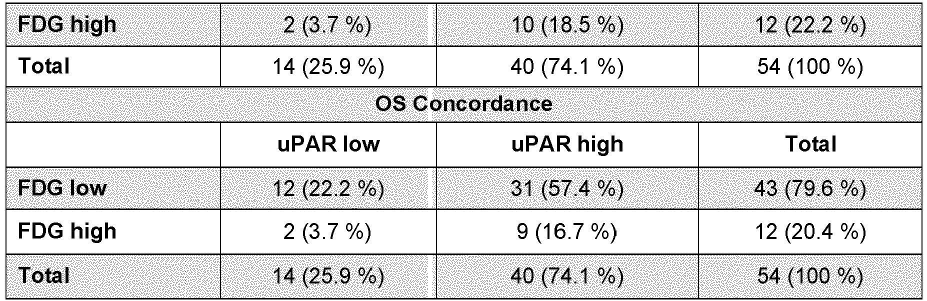

Overall, there was a significant difference between the groups in RFS and OS analysis (log-rank p=0.001). For RFS and OS, the concordant both high groups had a significantly poorer RFS compared to the concordant both low groups (p<0.0001). The group with discordant one low/one high uptake had an intermediate prognosis with a significantly more favorable prognosis compared to the both high group for both RFS and OS (p= 0.006 and p<0.0001), but inferior to the both low group, although not reaching significance (p=0.110 and p=0.069).

### Conclusion

This shows highly surprising that in a multivariate model that in addition to uPAR-PET included the routinely used 18F-FDG-PET scan, TNM stage and p16 status only uPAR-PET remained significant regarding prognosis expressed as RFS.

### Example 8 - Interrater Reliability

Interrater reliability in measurement of tumor SUVmax was good with an ICC of 0.835 (95% CI 0.713-0.905).

### Discussion of data

The main finding of the current prospective study was the ability of 68Ga-uPAR-PET/CT with 68Ga-NOTA-AE105 to predict RFS in HNSCC patients referred to curatively intended radiotherapy.

In univariate analysis 18F-FDG-SUVmax also predicted RFS, however, in a multivariate analysis including 68Ga-uPAR-SUVmax, 18F-FDG-SUVmax, TNM stage and p16 immunohistochemistry, only 68Ga-uPAR-SUVmax remained significant.

Accordingly, we demonstrated that a primary tumor 68Ga-uPAR-PET SUVmax cut-off could be established for identification of high- and low-risk groups in HNSCC patients referred to curatively intended radiotherapy. The PET parameter SUVmax is simple to obtain and the most frequently reported and most reproducible PET uptake metric in the literature (20).

The large proportion 8/16 (50%) of the non-HNC related deaths found in our study may explain why 68Ga-uPAR-PET was not able to predict OS. The poor general health status of many HNSCC patients is known to result in a high number of non-HNSCC deaths due to competing risks following tobacco and alcohol consumption (21). However, our study was not powered to evaluate 68Ga-uPAR-PET in predicting HNSCC-related deaths.

Since uPAR expression takes part in the tumor invasion and metastatic process *(12,14),* it is not surprising that high levels of uPAR-PET is related to relapse. Previous phase I clinical trials of 68Ga-uPAR-PET (*16,17,22*) as well as an array of preclinical studies (*13,23-26*) have demonstrated that our 68Ga-uPAR-PET indeed visualizes uPAR expression.

18F-FDG-PET SUVmax is the most common and well-characterized PET-uptake metric and a proposed prognostic marker in various cancers. Therefore, in our study we predefined 18F-FDG-PET SUVmax for comparison. For HNSCC, several studies have concluded that 18F-FDG-PET SUVmax does hold prognostic information, but results are inconsistent. Most of the studies are retrospective cohort studies and a concern has been that 18F-FDG is simply a surrogate marker of known clinical risk factors, especially tumor size (*10,11*). However, our results support the evidence of SUVmax being a significant predictor of patient outcome for both 68Ga-uPAR and 18F-FDG in univariate analysis.

18F-FDG is not tumor-specific and various image interpretation pitfalls exist due to physiological uptake and the complex anatomy of the head and neck (27). We found that 18F-FDG-PET SUVmax could predict OS but not RFS in the multivariate model. 68Ga-uPAR-PET remained significant regarding RFS in the multivariate model, but not 18F-FDG-PET, which demonstrates that the prognostic information obtained with 68Ga-uPAR is different from 18F-FDG-PET. The two tracers may be used for different purposes and complement each other in providing detailed non-invasive whole-tumor characterization (*28-30*). 68Ga-uPAR and 18F-FDG concordance could supply additional information to a future risk stratification of low (both low), intermediate (one low/one high) and high-risk patients (both high) for personalized treatment and follow-up strategies.

The more recent 18F-FDG-PET-uptake metrics metabolic tumor volume (MTV) and total lesion glycolysis (TLG) have shown promising prognostic results and inclusion of such parameters in future and larger phase studies could be of interest (31). Nonetheless, these parameters have several limitations and no consensus regarding volume segmentation and threshold have been established (31). For 68Ga-uPAR-PET prognostication we believe that SUVmax is the relevant metric for characterization of the most aggressive phenotype within the tumor and as a predictor of prognosis rather than a measure of volumes.

The current study represents a first proof-of-concept in a moderately sized study population. Larger future prospective (Phase III) studies are needed to establish the exact cut-off values that may also depend on the exact composition of the population. Nevertheless, with the current SUVmax cut-off point at 2.63, we identified the 25 % of patients with low risk of recurrence. Due to the considerable toxicity associated with chemoradiotherapy, initiatives to de-escalate the treatment for selected patients are being explored and 68Ga-uPAR may assist with a reliable identification of such low-risk patients (7).

Moreover, there is considerable variation in surveillance strategies following head-neck cancer treatment (32). Routine imaging is not standardized, and often patients request fewer follow-up visits (33). If results are validated, 68Ga-uPAR-PET may contribute to the development of risk stratified follow-up schedules.

In the past decades research in optimizing treatment for HNSCC patients has focused on the geometric precision of radiotherapy, but a shift towards biological precision has begun. The prognostic strength of 68Ga-uPAR-PET is the quantitative read out from tumor lesions and not a visual delineation, as some tumors may have low and almost no uptake. Accordingly, 68Ga-uPAR-PET will not replace 18F-FDG-PET as a diagnostic tool. In addition, 68Ga-uPAR-PET/CT may become an important companion diagnostics for selection of patients eligible for uPAR-targeted optical guided surgery using an uPAR-targeted optical probe or uPAR-targeted radionuclide therapy as well as for planning of external radiation therapy with customization of uPAR-targeted dose delivery of IMRT in patients with high tumor uptake (*23,34-37*).

### Conclusion

The current phase II clinical trial evaluating the prognostic impact of 68Ga-uPAR-PET/CT using 68Ga-NOTA-AE105 showed that 68Ga-uPAR-PET SUVmax can predict RFS in HNSCC patients referred to curatively intended radiotherapy. In a multivariate analysis including 68Ga-uPAR SUVmax, 18F-FDG SUVmax, TNM stage and p16 status only 68Ga-uPAR SUVmax remained significant for RFS. For OS, TNM stage and 18F-FDG SUVmax were significant.

### References

**1.** Chaturvedi AK, Engels EA, Pfeiffer RM, et al. Human papillomavirus and rising oropharyngeal cancer incidence in the United States. J Clin Oncol. 2011;29:4294-4301.
**2.** Mehanna H, Franklin N, Compton N, et al. Geographic variation in human papillomavirus-related oropharyngeal cancer: Data from 4 multinational randomized trials. Head Neck. 2016;38 Suppl 1:E1863-1869.
**3.** Albers AE, Qian X, Kaufmann AM, Coordes A. Meta analysis: HPV and p16 pattern determines survival in patients with HNSCC and identifies potential new biologic subtype. Sci Rep. 2017;7:16715.
**4.** O'Sullivan B, Huang SH, Su J, et al. Development and validation of a staging system for HPV-related oropharyngeal cancer by the International Collaboration on Oropharyngeal cancer Network for Staging (ICON-S): a multicentre cohort study. Lancet Oncol. 2016;17:440-451.
**5.** Jakobsen KK, Gronhoj C, Jensen DH, et al. Increasing incidence and survival of head and neck cancers in Denmark: a nation-wide study from 1980 to 2014. Acta Oncol. 2018;57:1143-1151.
**6.** Craig SG, Anderson LA, Schache AG, et al. Recommendations for determining HPV status in patients with oropharyngeal cancers under TNM8 guidelines: a two-tier approach. Br J Cancer. 2019;120:827-833.
**7.** Gillison ML, Trotti AM, Harris J, et al. Radiotherapy plus cetuximab or cisplatin in human papillomavirus-positive oropharyngeal cancer (NRG Oncology RTOG 1016): a randomised, multicentre, non-inferiority trial. Lancet. 2019;393:40-50.
**8.** Mehanna H, Robinson M, Hartley A, et al. Radiotherapy plus cisplatin or cetuximab in low-risk human papillomavirus-positive oropharyngeal cancer (De-ESCALaTE HPV): an open-label randomised controlled phase 3 trial. Lancet. 2019;393:51-60.
**9.** Burd EM. Human papillomavirus laboratory testing: the changing paradigm. Clin Microbiol Rev. 2016;29:291-319.
**10.** Clausen MM, Vogelius IR, Kjaer A, Bentzen SM. Multiple testing, cut-point optimization, and signs of publication bias in prognostic FDG-PET imaging studies of head and neck and lung cancer: A review and meta-analysis. Diagnostics (Basel). 2020;10:1030.
**11.** Rasmussen JH, Vogelius IR, Fischer BM, et al. Prognostic value of 18F-fludeoxyglucose uptake in 287 patients with head and neck squamous cell carcinoma. Head Neck. 2015;37:1274-1281.
**12.** Dass K, Ahmad A, Azmi AS, Sarkar SH, Sarkar FH. Evolving role of uPA/uPAR system in human cancers. Cancer Treat Rev. 2008;34:122-136.
**13.** Persson M, Kjaer A. Urokinase-type plasminogen activator receptor (uPAR) as a promising new imaging target: potential clinical applications. Clin Physiol Funct Imaging. 2013;33:329-337.
**14.** Mekkawy AH, Pourgholami MH, Morris DL. Involvement of urokinase-type plasminogen activator system in cancer: an overview. Med Res Rev. 2014;34:918-956.
**15.** Skovgaard D, Persson M, Brandt-Larsen M, et al. Safety, dosimetry, and tumor detection ability of (68)Ga-NOTA-AE105: First-in-human study of a novel radioligand for uPAR PET imaging. J Nucl Med. 2017;58:379-386.
**16.** Skovgaard D, Persson M, Kjaer A. Urokinase plasminogen activator receptor-PET with (68)Ga-NOTA-AE105: First clinical experience with a novel PET ligand. PET Clin. 2017;12:311-319.
**17.** Persson M, Skovgaard D, Brandt-Larsen M, et al. First-in-human uPAR PET: Imaging of cancer aggressiveness. Theranostics. 2015;5:1303-1316.
**18.** Guidelines D. www.dahanca.dk/guidelines (In Danish), Accessed on October 1't 2020.
**19.** Budczies J, Klauschen F, Sinn BV, et al. Cutoff Finder: a comprehensive and straightforward web application enabling rapid biomarker cutoff optimization. PLoS One. 2012;7.
**20.** Wahl RL, Jacene H, Kasamon Y, Lodge MA. From RECIST to PERCIST: Evolving considerations for PET response criteria in solid tumors. J Nucl Med. 2009;50 Suppl 1:1225-150S.
**21.** Gillison ML, Zhang Q, Jordan R, et al. Tobacco smoking and increased risk of death and progression for patients with p16-positive and p16-negative oropharyngeal cancer. J Clin Oncol. 2012;30:2102-2111.
**22.** Skovgaard D, Persson M, Kjaer A. Imaging of prostate cancer using rokinase-Type Plasminogen Activator Receptor PET. PET Clin. 2017;12:243-255.
**23.** Persson M, Madsen J, Ostergaard S, et al. Quantitative PET of human urokinase-type plasminogen activator receptor with 64Cu-DOTA-AE105: implications for visualizing cancer invasion. J Nucl Med. 2012;53:138-145.
**24.** Persson M, Hosseini M, Madsen J, et al. Improved PET imaging of uPAR expression using new (64)Cu-labeled cross-bridged peptide ligands: comparative in vitro and in vivo studies. Theranostics. 2013;3:618-632.
**25.** Persson M, Liu H, Madsen J, Cheng Z, Kjaer A. First (18)F-labeled ligand for PET imaging of uPAR: in vivo studies in human prostate cancer xenografts. Nucl Med Biol. 2013;40:618-624.
**26.** Persson M, El Ali HH, Binderup T, et al. Dosimetry of 64Cu-DOTA-AE105, a PET tracer for uPAR imaging. Nucl Med Biol. 2014;41:290-295.
**27.** Purohit BS, Ailianou A, Dulguerov N, Becker CD, Ratib O, Becker M. FDG-PET/CT pitfalls in oncological head and neck imaging. Insights Imaging. 2014;5:585-602.
**28.** Marcu LG, Reid P, Bezak E. The promise of novel biomarkers for head and neck cancer from an imaging perspective. Int J Mol Sci. 2018;19:2511.
**29.** Song IH, Noh Y, Kwon J, et al. Immuno-PET imaging based radioimmunotherapy in head and neck squamous cell carcinoma model. Oncotarget. 2017;8:92090-92105.
**30.** Christensen A, Kiss K, Lelkaitis G, et al. Urokinase-type plasminogen activator receptor (uPAR), tissue factor (TF) and epidermal growth factor receptor (EGFR): tumor expression patterns and prognostic value in oral cancer. BMC Cancer. 2017;17:572.
**31.** Rijo-Cedeno J, Mucientes J, Seijas Marcos S, et al. Adding value to tumor staging in head and neck cancer: The role of metabolic parameters as prognostic factors. Head Neck. 2021;43:2477-2487.
**32.** Wong WL. PET-CT for staging and detection of recurrence of head and neck cancer. Semin Nucl Med. 2021;51:13-25.
**33.** Trinidade A, Kothari P, Andreou Z, Hewitt RJ, O'Flynn P. Follow-up in head and neck cancer: patients' perspective. Int J Health Care Qual Assur. 2012;25:145-149.
**34.** Christensen A, Juhl K, Persson M, et al. uPAR-targeted optical near-infrared (NIR) fluorescence imaging and PET for image-guided surgery in head and neck cancer: proof-of-concept in orthotopic xenograft model. Oncotarget. 2017;8:15407-15419.
**35.** Ling CC, Humm J, Larson S, et al. Towards multidimensional radiotherapy (MD-CRT): biological imaging and biological conformality. Int J Radiat Oncol Biol Phys. 2000;47:551-560.
**36.** Atallah I, Milet C, Henry M, et al. Near-infrared fluorescence imaging-guided surgery improves recurrence-free survival rate in novel orthotopic animal model of head and neck squamous cell carcinoma. Head Neck. 2016;38 Suppl 1:E246-255.
**37.** Persson M, Madsen J, Ostergaard S, Ploug M, Kjaer A. 68Ga-labeling and in vivo evaluation of a uPAR binding DOTA- and NODAGA-conjugated peptide for PET imaging of invasive cancers. Nucl Med Biol. 2012;39:560-569.

## Claims

1. A positron-emitting imaging agent for use in the prognostication of relapse-free survival (RFS) and/or overall survival (OS) of a head and neck squamous cell carcinoma (HNSCC) in a human patient by PET imaging of the cancer,
wherein said imaging agent comprises a uPAR binding peptide coupled via the chelating agent NOTA or DOTA to the radionuclide 68Ga or 64Cu; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof;
wherein the uPAR binding variant is selected from the group consisting of
• (D-Asp)-([beta]-cyclohexyl-L-alanine)-(Phe)-(D-Ser)-(D-Arg)-(Tyr)-(Leu)-(Trp)-(Ser),
• (Ser)-(Leu)-([beta]-cyclohexyl-L-alanine)-(Phe)-(D-Ser)-(Gln)-(Tyr)(Leu)-(Trp)-(Ser),
• (D-Glu)-([beta]-cyclohexyl-L-alanine)-(Phe)-(D-Ser)-(D-Tyr)-(Tyr)-(Leu)-(Trp)-(Ser),
• (Asp)-([beta]-cyclohexyl-L-alanine)-(Phe)-(D-Ser)-(D-Arg)-(Tyr)-(Leu)-(Trp)-(Ser),
• (Asp)-([beta]-cyclohexyl-L-alanine)-(Phe)-(Ser)-(D-Arg)-(Tyr)-Leu)-(Trp)-(Ser),
• (D-Asp)-([beta]-cyclohexyl-L-alanine)-(Phe)-(Ser)-(D-Arg)-(Tyr)-Leu)-(Trp)-(Ser),
• (D-Thr)-([beta]-cyclohexyl-L-alanine)-(Phe)-(D-Ser)-(D-Arg)-(Tyr)-(Leu)-(Trp)-(Ser),
• (D-Asp)-([beta]-cyclohexyl-L-alanine)-(Phe)-(D-Ser)-(D-Arg)-(Tyr)-(Leu)-([beta]-2-naphthyl-L-alanine)-(Ser),
• (Asp)-([beta]-cyclohexyl-L-alanine)-(Phe)-(D-Ser)-(Arg)-(Tyr)-(Leu)-(Trp)-(Ser),
• (Asp)-([beta]-cyclohexyl-L-alanine)-(Phe)-(D-Ser)-(D-Arg)-(Tyr)-(Leu)([beta]-1-naphthyl-L-alanine)-(Ser),
• (D-Glu)-([beta]-cyclohexyl-L-alanine)-(Phe)-(D-Ser)-(Tyr)-(Tyr)-(Leu)-(Trp)-(Ser),
• (Asp)-([beta]-cyclohexyl-L-alanine)-(Phe)-(D-Ser)-(D-Arg)-(Leu)-(Leu)-(Trp)-(D-His),
• (Asp)-([beta]-cyclohexyl-L-alanine)-(Phe)-(D-Ser)-(D-Arg)-([beta]-cyclohexyl-L-alanine)-(Leu)-(Trp)-(Ile),
• (Asp)-([beta]-cyclohexyl-L-alanine)-(Phe)-(D-Ser)-(D-Arg)-(Tyr)-(Leu)([beta]-1-naphthyl-L-alanine)-(D-His),
• (Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
• (Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
• (Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine), and
• (Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(Ile);
- wherein a SUVmax and/or SUVmean quantification level above a threshold level is indicative of a poor Relapse-free survival (RFS) prognosis and/or poor overall survival (OS) prognosis; and
- wherein a SUVmax and/or SUVmean quantification level equal to or below a threshold level is indicative of good Relapse-free survival (RFS) prognosis and/or good overall survival (OS) prognosis.

2. The imaging agent for use according to claim 1, wherein the peptide is (D-Asp)-([beta]-cyclohexyl-L-alanine)-(Phe)-(D-Ser)-(D-Arg)-(Tyr)-(Leu)-(Trp)-(Ser).

3. The imaging agent for use according to any of the preceding claims, having the formula

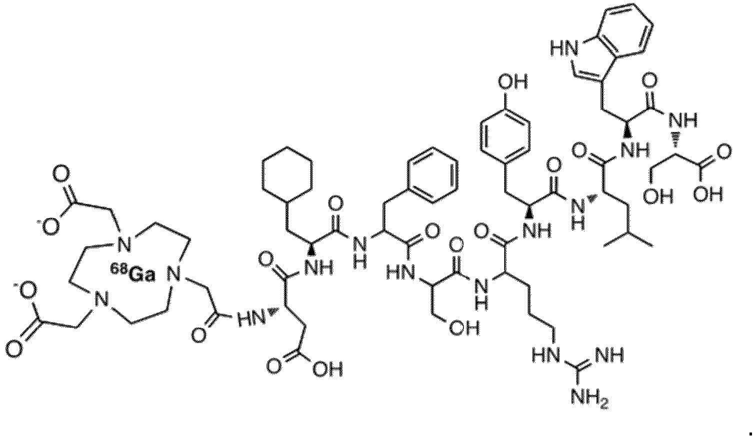

4. The imaging agent for use according to any of the preceding claims 1-2, having the formula

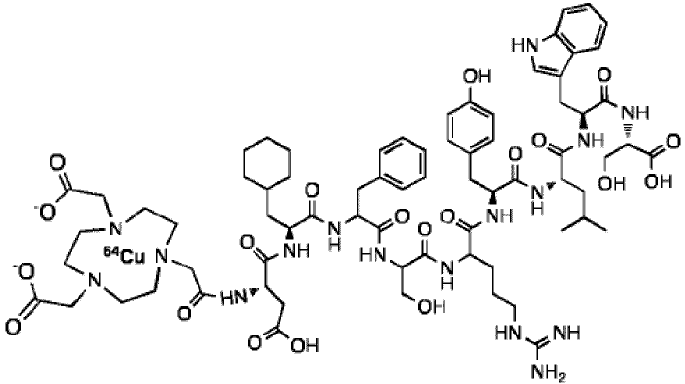

5. The imaging agent for use according to any of the preceding claims 1-2, having the formula

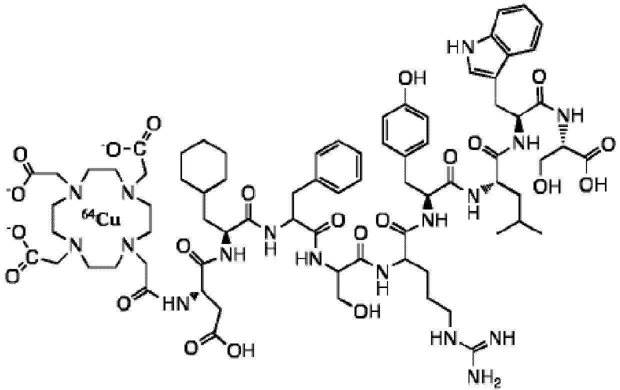

6. The imaging agent for use according to any of the preceding claims, wherein the prognosis is relapse-free survival (RFS).

7. The imaging agent for use according to any of the preceding claims, wherein the imaging agent is to be administered in a dose of 10-500 MBq followed by PET scanning 10 min to 24 hours after the imaging agent has been administered, and quantification through SUVmax and/or SUVmean.

8. The imaging agent for use according to any of the preceding claims, wherein the imaging agent is to be administered in a dose of 20-400 MBq, such as 50-400 MBq, such as 70-300 MBq, such as 100-300 MBq or such as 100-300 MBq.

9. The imaging agent for use according to any of the preceding claims, wherein the imaging agent is to be administered in a dose of 70-300 MBq.

10. The imaging agent for use according to any of the preceding claims,
∘ wherein a SUVmax and/or SUVmean quantification level above a threshold level is indicative of a poor Relapse-free survival (RFS) prognosis; and
∘ wherein a SUVmax and/or SUVmean quantification level equal to or below a threshold level is indicative of good Relapse-free survival (RFS) prognosis.

11. The imaging agent for use according to any of the preceding claims, wherein said SUVmax and/or SUVmean threshold level is in the range 1-4, such as in the range 2-4 preferably in the range 2-3, such as 2.4-2.8.

12. The imaging agent for use according to any of the preceding claims, wherein said threshold level is determined by using a cut-off finding method to obtain a split in a Kaplan-Meier plot (log-rank test) and the corresponding hazard ratios (HRs).

13. The imaging agent for use according to any of the preceding claims, wherein said human patient has been referred to curatively intended radiotherapy.

14. The imaging agent for use according to any of the preceding claims, wherein the HNSCC is located in the pharynx, larynx or oral cavity.

## Patentansprüche

1. Positronen emittierendes Abbildungsmittel zur Verwendung bei der Prognose des rezidivfreien Überlebens (RFS) und/oder des Gesamtüberlebens (OS) eines Kopf- und Nackenplattenepithelkarzinoms (HNSCC) bei einem menschlichen Patienten durch PET-Abbildung des Krebses,
wobei das Abbildungsmittel ein uPAR-bindendes Peptid umfasst, das über den Chelatbildner NOTA oder DOTA an das Radionuklid 68Ga oder 64Cu gekoppelt ist; und
wobei das uPAR-bindende Peptid (D-Asp)-([beta]-Cyclohexyl-L-alanin)-(Phe)-(D-Ser)-(D-Arg)-(Tyr)-(Leu)-(Trp)-(Ser) oder eine uPAR-bindende Variante davon ist;
wobei die uPAR-bindende Variante ausgewählt ist aus der Gruppe, bestehend aus
• (D-Asp)-([beta]-Cyclohexyl-L-alanin)-(Phe)-(D-Ser)-(D-Arg)-(Tyr)-(Leu)-(Trp)-(Ser),
• (Ser)-(Leu)-([beta]-Cyclohexyl-L-alanin)-(Phe)-(D-Ser)-(Gln)-(Tyr)(Leu)-(Trp)-(Ser),
• (D-Glu)-([beta]-Cyclohexyl-L-alanin)-(Phe)-(D-Ser)-(D-Tyr)-(Tyr)-(Leu)-(Trp)-(Ser),
• (Asp)-([beta]-Cyclohexyl-L-alanin)-(Phe)-(D-Ser)-(D-Arg)-(Tyr)-(Leu)-(Trp)-(Ser),
• (Asp)-([beta]-Cyclohexyl-L-alanin)-(Phe)-(Ser)-(D-Arg)-(Tyr)-Leu)-(Trp)-(Ser),
• (D-Asp)-([beta]-Cyclohexyl-L-alanin)-(Phe)-(Ser)-(D-Arg)-(Tyr)-Leu)-(Trp)-(Ser),
• (D-Thr)-([beta]-Cyclohexyl-L-alanin)-(Phe)-(D-Ser)-(D-Arg)-(Tyr)-(Leu)-(Trp)-(Ser),
• (D-Asp)-([beta]-Cyclohexyl-L-alanin)-(Phe)-(D-Ser)-(D-Arg)-(Tyr)-(Leu)-([beta]-2-Naphthyl-L-alanin)-(Ser),
• (Asp)-([beta]-Cyclohexyl-L-alanin)-(Phe)-(D-Ser)-(Arg)-(Tyr)-(Leu)-(Trp)-(Ser),
• (Asp)-([beta]-Cyclohexyl-L-alanin)-(Phe)-(D-Ser)-(D-Arg)-(Tyr)-(Leu)([beta]-1-Naphthyl-L-alanin)-(Ser),
• (D-Glu)-([beta]-Cyclohexyl-L-alanin)-(Phe)-(D-Ser)-(Tyr)-(Tyr)-(Leu)-(Trp)-(Ser),
• (Asp)-([beta]-Cyclohexyl-L-alanin)-(Phe)-(D-Ser)-(D-Arg)-(Leu)-(Leu)-(Trp)-(D-His),
• (Asp)-([beta]-Cyclohexyl-L-alanin)-(Phe)-(D-Ser)-(D-Arg)-([beta]-Cyclohexyl-L-alanin)-(Leu)-(Trp)-(Ile),
• (Asp)-([beta]-Cyclohexyl-L-alanin)-(Phe)-(D-Ser)-(D-Arg)-(Tyr)-(Leu)([beta]-1-Naphthyl-L-alanin)-(D-His),
• (Asp)-([beta]-Cyclohexyl-L-alanin)-(Phe)-(D-Ser)-(D-Arg)-(N-(2,3-dimethoxybenzyl)glycin)-(D-Phe)-(N-(3-Indolylethyl)glycin)-(N-(2-methoxyethyl)glycin),
• (Asp)-([beta]-Cyclohexyl-L-alanin)-(Phe)-(D-Ser)-(D-Arg)-(N-(2,3-Dimethoxybenzyl)glycin)-(D-Phe)-(N-Benzylglycin)-(N-(2[beta]thoxyethyl)glycin),
• (Asp)-([beta]-Cyclohexyl-L-alanin)-(Phe)-(D-Ser)-(D-Arg)-(N-(2,3-dimethoxybenzyl)glycin)-(D-Phe)-(N-(Methylnaphthalyl)glycin)-(N-(2-Methoxyethyl)glycin), and
• (Asp)-([beta]-Cyclohexyl-L-alanin)-(Phe)-(D-Ser)-(D-Arg)-(N-(2,3-Dimethoxybenzyl)glycin)-(D-Phe)-(N-(2,3-Dimethoxybenzyl)glycin)-(Ile);
- wobei ein SUVmax- und/oder ein SUVmean-Quantifizierungsniveau oberhalb eines Schwellenniveaus auf eine schlechte Prognose des rezidivfreien Überlebens (RFS) und/oder eine schlechte Prognose des Gesamtüberlebens (OS) hinweist; und
- wobei ein SUVmax- und/oder ein SUVmean-Quantifizierungsniveau, das einem Schwellenniveau entspricht oder darunter liegt, auf eine gute Prognose des rezidivfreien Überlebens (RFS) und/oder eine gute Prognose des Gesamtüberlebens (OS) hinweist.

2. Abbildungsmittel zur Verwendung nach Anspruch 1, wobei das Peptid (D-Asp)-([beta]-Cyclohexyl-L-alanin)-(Phe)-(D-Ser)-(D-Arg)-(Tyr)-(Leu)-(Trp)-(Ser) ist.

3. Abbildungsmittel zur Verwendung nach einem der vorstehenden Ansprüche, aufweisend die Formel

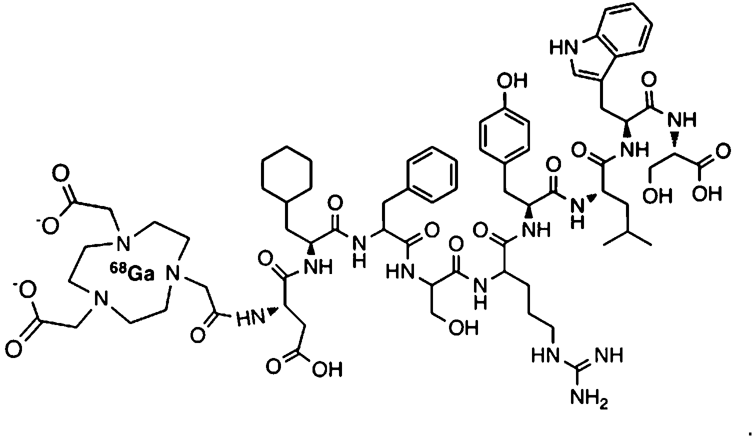

4. Abbildungsmittel zur Verwendung nach einem der vorstehenden Ansprüche 1-2, aufweisend die Formel

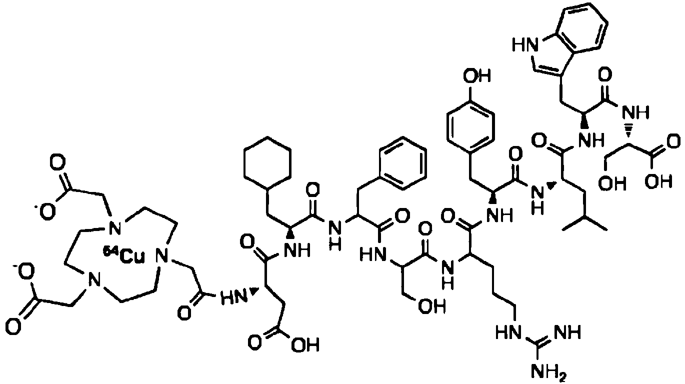

5. Abbildungsmittel zur Verwendung nach einem der vorstehenden Ansprüche 1-2, aufweisend die Formel

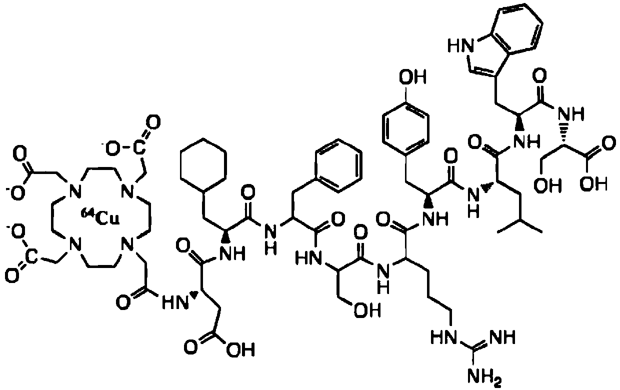

6. Abbildungsmittel zur Verwendung nach einem der vorstehenden Ansprüche, wobei die Prognose rezidivfreies Überleben (RFS) ist.

7. Abbildungsmittel zur Verwendung nach einem der vorstehenden Ansprüche, wobei das Abbildungsmittel in einer Dosis von 10-500 MBq zu verabreichen ist, gefolgt von einem PET-Scan 10 Minuten bis 24 Stunden nach der Verabreichung des Abbildungsmittels und einer Quantifizierung durch SUVmax und/oder SUVmean.

8. Abbildungsmittel zur Verwendung nach einem der vorstehenden Ansprüche, wobei das Abbildungsmittel in einer Dosis von 20-400 MBq, wie 50-400 MBq, wie 70-300 MBq, wie 100-300 MBq oder wie 100-300 MBq zu verabreichen ist.

9. Abbildungsmittel zur Verwendung nach einem der vorstehenden Ansprüche, wobei das Abbildungsmittel in einer Dosis von 70-300 MBq zu verabreichen ist.

10. Abbildungsmittel zur Verwendung nach einem der vorstehenden Ansprüche,
o wobei ein SUVmax- und/oder ein SUVmean-Quantifizierungsniveau oberhalb eines Schwellenniveaus auf eine schlechte Prognose des rezidivfreien Überlebens (RFS) hinweist; und
o wobei ein SUVmax- und/oder ein SUVmean-Quantifizierungsniveau, das einem Schwellenniveau entspricht oder darunter liegt, auf eine gute Prognose des rezidivfreien Überlebens (RFS) hinweist.

11. Abbildungsmittel zur Verwendung nach einem der vorstehenden Ansprüche, wobei das SUVmax- und/oder SUVmean-Schwellenniveau im Bereich 1-4, wie im Bereich 2-4, vorzugsweise im Bereich 2-3, wie 2,4-2,8, liegt.

12. Abbildungsmittel zur Verwendung nach einem der vorstehenden Ansprüche, wobei das Schwellenniveau durch Verwenden eines Obergrenzenfindungsverfahrens bestimmt wird, um eine Aufteilung in einem Kaplan-Meier-Diagramm (Log-Rank-Test) und die entsprechenden Risikoverhältnisse (HRs) zu erhalten.

13. Abbildungsmittel zur Verwendung nach einem der vorstehenden Ansprüche, wobei der menschliche Patient zu einer kurativ intendierten Strahlentherapie überwiesen wurde.

14. Abbildungsmittel zur Verwendung nach einem der vorstehenden Ansprüche, wobei sich das HNSCC in dem Rachen, dem Kehlkopf oder der Mundhöhle befindet.

## Revendications

1. Agent d'imagerie à émission de positons pour utilisation dans le pronostic de survie sans récidive (RFS) et/ou de survie globale (OS) d'un carcinome à cellules squameuses de la tête et du cou (HNSCC) chez un patient humain par imagerie PET du cancer,
dans lequel ledit agent d'imagerie comprend un peptide de liaison à uPAR couplé par l'intermédiaire de l'agent chélateur NOTA ou DOTA au radionucléide 68Ga ou 64Cu ; et
dans lequel le peptide de liaison à uPAR est (D-Asp)-([bêta]-cyclohexyl-L-alanine)-(Phe)-(D-Ser)-(D-Arg)-(Tyr)-(Leu)-(Trp)-(Ser) ou un variant de liaison à uPAR de celui-ci ;
dans lequel le variant de liaison à uPAR est choisi dans le groupe constitué par
• (D-Asp)-([bêta]-cyclohexyl-L-alanine)-(Phe)-(D-Ser)-(D-Arg)-(Tyr)-(Leu)-(Trp)-(Ser),
• (Ser)-(Leu)-([bêta]-cyclohexyl-L-alanine)-(Phe)-(D-Ser)-(Gln)-(Tyr)(Leu)-(Trp)-(Ser),
• (D-Glu)-([bêta]-cyclohexyl-L-alanine)-(Phe)-(D-Ser)-(D-Tyr)-(Tyr)-(Leu)-(Trp)-(Ser),
• (Asp)-([bêta]-cyclohexyl-L-alanine)-(Phe)-(D-Ser)-(D-Arg)-(Tyr)-(Leu)-(Trp)-(Ser),
• (Asp)-([bêta]-cyclohexyl-L-alanine)-(Phe)-(Ser)-(D-Arg)-(Tyr)-Leu)-(Trp)-(Ser),
• (D-Asp)-([bêta]-cyclohexyl-L-alanine)-(Phe)-(Ser)-(D-Arg)-(Tyr)-Leu)-(Trp)-(Ser),
• (D-Thr)-([bêta]-cyclohexyl-L-alanine)-(Phe)-(D-Ser)-(D-Arg)-(Tyr)-(Leu)-(Trp)-(Ser),
• (D-Asp)-([bêta]-cyclohexyl-L-alanine)-(Phe)-(D-Ser)-(D-Arg)-(Tyr)-(Leu)-([bêta]-2-naphtyl-L-alanine)-(Ser),
• (Asp)-([bêta]-cyclohexyl-L-alanine)-(Phe)-(D-Ser)-(Arg)-(Tyr)-(Leu)-(Trp)-(Ser),
• (Asp)-([bêta]-cyclohexyl-L-alanine)-(Phe)-(D-Ser)-(D-Arg)-(Tyr)-(Leu)([bêta]-1-naphtyl-L-alanine)-(Ser),
• (D-Glu)-([bêta]-cyclohexyl-L-alanine)-(Phe)-(D-Ser)-(Tyr)-(Tyr)-(Leu)-(Trp)-(Ser),
• (Asp)-([bêta]-cyclohexyl-L-alanine)-(Phe)-(D-Ser)-(D-Arg)-(Leu)-(Leu)-(Trp)-(D-His),
• (Asp)-([bêta]-cyclohexyl-L-alanine)-(Phe)-(D-Ser)-(D-Arg)-([bêta]-cyclohexyl-L-alanine)-(Leu)-(Trp)-(Ile),
• (Asp)-([bêta]-cyclohexyl-L-alanine)-(Phe)-(D-Ser)-(D-Arg)-(Tyr)-(Leu)([bêta]-1-naphtyl-L-alanine)-(D-His),
• (Asp)-([bêta]-cyclohexyl-L-alanine)-(Phe)-(D-Ser)-(D-Arg)-(N-(2,3-diméthoxybenzyl)glycine)-(D-Phe)-(N-(3-indolyléthyl)glycine)-(N-(2-méthoxyéthyl)glycine),
• (Asp)-([bêta]-cyclohexyl-L-alanine)-(Phe)-(D-Ser)-(D-Arg)-(N-(2,3-diméthoxybenzyl)glycine)-(D-Phe)-(N-benzylglycine)-(N-(2[bêta]thoxyéthyl)glycine),
• (Asp)-([bêta]-cyclohexyl-L-alanine)-(Phe)-(D-Ser)-(D-Arg)-(N-(2,3-diméthoxybenzyl)glycine)-(D-Phe)-(N-(méthylnaphtalyl)glycine)-(N-(2-méthoxyéthyl)glycine), et
• (Asp)-([bêta]-cyclohexyl-L-alanine)-(Phe)-(D-Ser)-(D-Arg)-(N-(2,3-diméthoxybenzyl)glycine)-(D-Phe)-(N-(2,3-diméthoxybenzyl)glycine)-(Ile) ;
- dans lequel un niveau de quantification SUVmax et/ou SUVmoyenne supérieur à un niveau seuil est indicatif d'un mauvais pronostic de survie sans récidive (RFS) et/ou d'un mauvais pronostic de survie globale (OS) ; et
- dans lequel un niveau de quantification SUVmax et/ou SUVmoyenne égal ou inférieur à un niveau seuil est indicatif d'un bon pronostic de survie sans récidive (RFS) et/ou d'un bon pronostic de survie globale (OS).

2. Agent d'imagerie pour utilisation selon la revendication 1, dans lequel le peptide est (D-Asp)-([bêta]-cyclohexyl-L-alanine)-(Phe)-(D-Ser)-(D-Arg)-(Tyr)-(Leu)-(Trp)-(Ser).

3. Agent d'imagerie pour utilisation selon l'une quelconque des revendications précédentes, ayant la formule

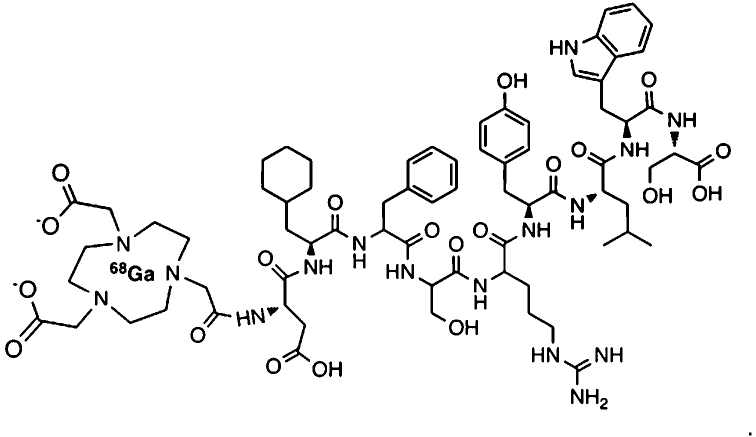

4. Agent d'imagerie pour utilisation selon l'une quelconque des revendications 1-2 précédentes, ayant la formule

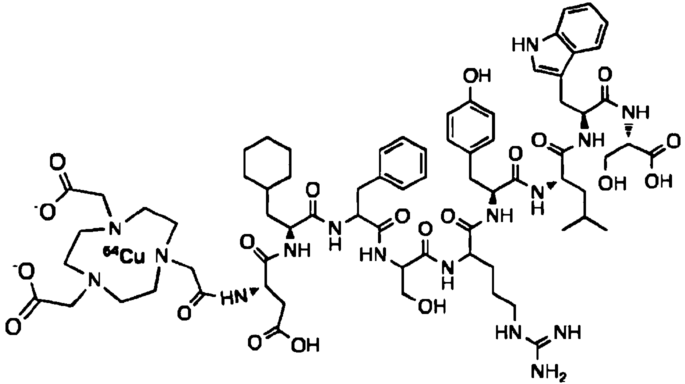

5. Agent d'imagerie pour utilisation selon l'une quelconque des revendications 1-2 précédentes, ayant la formule

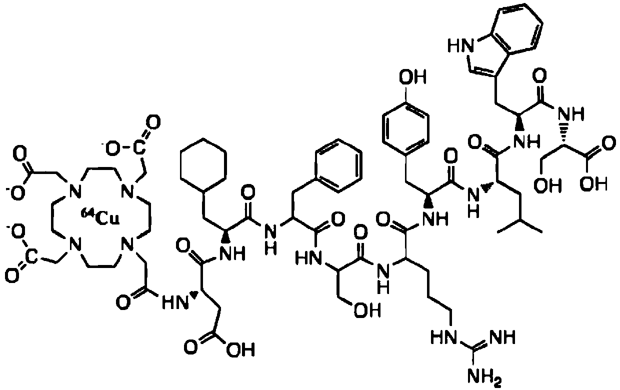

6. Agent d'imagerie pour utilisation selon l'une quelconque des revendications précédentes, dans lequel le pronostic est la survie sans récidive (RFS).

7. Agent d'imagerie pour utilisation selon l'une quelconque des revendications précédentes, dans lequel l'agent d'imagerie doit être administré à une dose de 10-500 MBq, suivie d'un examen par PET 10 minutes à 24 heures après l'administration de l'agent d'imagerie, et d'une quantification par SUVmax et/ou SUVmoyenne.

8. Agent d'imagerie pour utilisation selon l'une quelconque des revendications précédentes, dans lequel l'agent d'imagerie doit être administré à une dose de 20-400 MBq, par exemple 50-400 MBq, par exemple 70-300 MBq, par exemple 100-300 MBq, ou par exemple 100-300 MBq.

9. Agent d'imagerie pour utilisation selon l'une quelconque des revendications précédentes, dans lequel l'agent d'imagerie doit être administré à une dose de 70-300 MBq.

10. Agent d'imagerie pour utilisation selon l'une quelconque des revendications précédentes,
o dans lequel un niveau de quantification SUVmax et/ou SUVmoyenne supérieur à un niveau seuil est indicatif d'un mauvais pronostic de survie sans récidive (RFS) ; et
o dans lequel un niveau de quantification SUVmax et/ou SUVmoyenne égal ou inférieur à un niveau seuil est indicatif d'un bon pronostic de survie sans récidive (RFS).

11. Agent d'imagerie pour utilisation selon l'une quelconque des revendications précédentes, dans lequel ledit niveau seuil SUVmax et/ou SUVmoyenne est dans la plage de 1-4, par exemple dans la plage de 2-4, de préférence dans la plage de 2-3, par exemple de 2.4-2.8.

12. Agent d'imagerie pour utilisation selon l'une quelconque des revendications précédentes, dans lequel ledit niveau seuil est déterminé en utilisant une méthode de recherche de seuil permettant d'obtenir une séparation dans un diagramme de Kaplan-Meier (test du log-rank) et les rapports de risque (HR) correspondants.

13. Agent d'imagerie pour utilisation selon l'une quelconque des revendications précédentes, dans lequel ledit patient humain a été orienté vers une radiothérapie à visée curative.

14. Agent d'imagerie pour utilisation selon l'une quelconque des revendications précédentes, dans lequel le HNSCC est situé dans le pharynx, le larynx ou la cavité buccale.
